# EUROPEAN PATENT SPECIFICATION

(11) **EP 3 852 616 B1**
(45) Date of publication and mention of the grant of the patent: **26.08.2026**
(21) Application number: 19862618.6
(22) Date of filing: 17.09.2019
(51) Int. Cl.: A61B 5/02, A61B 5/0205, A61B 5/024, A61B 5/08, A61B 5/113, A61B 5/145, A61B 5/00

(54) **SYSTEMS AND METHODS FOR ANALYSIS OF SLEEP DISORDERED BREATHING EVENTS**
SYSTEME UND VERFAHREN ZUR ANALYSE VON ATEMSTÖRUNGEN WÄHREND DES SCHLAFES
SYSTÈMES ET MÉTHODES D'ANALYSE D'ÉVÉNEMENTS DE TROUBLES RESPIRATOIRES DU SOMMEIL

(30) Priority: 17.09.2018 US 201862732490 P
(43) Date of publication of application: 28.07.2021
(73) Proprietor: Mycardio LLC, Denver, CO 80206 (US)
(72) Inventor: HILMISSON, Hugi, Denver, Colorado 80220 (US)
(74) Representative: Page White Farrer
(86) International application number: PCT/US2019/051472
(87) International publication number: WO 2020/061014

(56) References cited:
- US-A1- 2005 115 561
- US-A1- 2005 115 561
- US-A1- 2007 173 728
- US-A1- 2007 173 728
- US-A1- 2007 213 621
- US-A1- 2007 213 621
- THOMAS PENZEL ET AL: "Modulations of Heart Rate, ECG, and Cardio-Respiratory Coupling Observed in Polysomnography", FRONTIERS IN PHYSIOLOGY, vol. 7, 25 October 2016 (2016-10-25), XP055723417, DOI: 10.3389/fphys.2016.00460
- MAGNUSDOTTIR SOLVEIG ET AL: "Ambulatory screening tool for sleep apnea: analyzing a single-lead electrocardiogram signal (ECG)", SLEEP AND BREATHING - SCHLAF UND ATMUNG, DRUCKBILD, TITISEE-NEUSTADT, DE, vol. 22, no. 2, 7 September 2017 (2017-09-07), pages 421 - 429, XP036489294, ISSN: 1520-9512, [retrieved on 20170907], DOI: 10.1007/S11325-017-1566-6
- ANONYMOUS: "Cardiopulmonary Coupling and its relationship to PSG", SLEEPIMAGE, 25 February 2018 (2018-02-25), pages 1 - 4, XP055799695, Retrieved from the Internet <URL:https://web.archive.org/web/20180225212748/http://www.sleepimage.com:80/getmedia/97ec44ba-0693-4884-aa05-01884edc7931/Cardiopulmonary-Coupling-and-its-Relationship-with-PSG.aspx> [retrieved on 20191031]
- CHEN ET AL.: "Assessment of sleep quality using cardiopulmonary coupling analysis in patients with Parkinson's disease", 31 May 2018 (2018-05-31), XP055693749, Retrieved from the Internet <URL:https://www.ncbi.nlm.nih.gov/pmc/articles/PMC5943733> [retrieved on 20191031]
- THOMAS ET AL.: "An Electrocardiogram-Based Technique to Assess Cardiopulmonary Coupling During Sleep", 30 September 2005 (2005-09-30), pages 1151 - 1161, XP055693752, Retrieved from the Internet <URL:https://dbiom.org/files/publications/Peng_ElectrocardiogramTechniqueCardiopulmonaryCouplingSleep.pdf> [retrieved on 20191031]

## Description

### BACKGROUND

### Technical Field

The present disclosure relates to sleep analysis, and in particular to analyzing sleep disordered breathing events during sleep.

### Description of Related Art

At least five percent of the general population suffers from medically significant sleep disorders, with the most common being sleep-disordered breathing (also known as sleep apnea). As a major public health concern, sleep disorders contribute to excessive daytime sleepiness and the associated risks of driving accidents, hypertension, heart attacks, strokes, depression, and/or attention deficit disorders. The prevalence of sleep disorders is much higher (exceeding thirty percent) in select populations, such as individuals having obesity, congestive heart failure, diabetes, and/or renal failure.

Conventional diagnostic systems exist for detecting sleep disordered breathing which provide simple, inexpensive, and repeatable measures of the presence and impact of a variety of sleep disruptive stimuli (such as noise, pain, drugs, mood disorders, disordered breathing) on sleep state physiology and stability. An example of a conventional sleep diagnostic system is a full polysomnograph. Polysomnography is considered the reference standard for detection and quantification of sleep-disordered breathing, and includes sleep staging, scoring of respiratory abnormality (e.g., apneas, hypopneas, flow-limitation, periodic breathing, and desaturation episodes), and limb movements. Various markers of sleep disorder severity include the sleep fragmentation index, the apnea-hypopnea index, the respiratory disturbance index, an arousal frequency or index, and/or the oxygen desaturation index. For example, Apnea-Hypopnea Index (AHI) is used to determine how many times each hour an apnea or hypopnea event has occurred. The apnea-hypopnea index is interpreted as a severity index by comparing the score to defined thresholds, with the implication being that a higher prevalence indicates a more severe condition.

US 2007/213624 A1 (Reisfeld, Daniel et al., published 13 September 2007) reports "Detection of Heart Failure Using a Photoplethysmograph").

There is interest in further developing and improving technologies for analyzing sleep disordered breathing events for type, prevalence, and/or severity.

### SUMMARY

The present disclosure provides methods and systems for sleep assessment. In accordance with aspects of the present disclosure, the method includes accessing recorded oxygen saturation data including oxygen saturation measurements obtained from a person over a time period, determining disordered breathing events for the person over the time period based on the oxygen saturation data, accessing recorded cardiopulmonary coupling data obtained from the person during sleep, wherein the cardiopulmonary coupling data is correlated in time with the oxygen saturation data over the time period, and determining a type of cardiopulmonary coupling event for each of the disordered breathing events which were determined based on the oxygen saturation data, based on the cardiopulmonary coupling data corresponding in time to the disordered breathing events which were determined based on the oxygen saturation data.

In various embodiments of the method, the method includes determining based on the oxygen saturation data that the events are sleep disordered breathing events.

In various embodiments of the method, determining the disordered breathing events includes identifying a start of a potential disordered breathing event by a decrease between a first oxygen saturation measurement and a second oxygen saturation measurement in the oxygen saturation data, where the second oxygen saturation measurement is temporally after the first oxygen saturation measurement, and validating the potential disordered breathing event by at least one condition.

In various embodiments of the method, the condition includes at least one of successive oxygen saturation measurements remaining unchanged for a predetermined plateau duration, successive oxygen saturation measurements increasing for a predetermined increase duration, reaching a predetermined duration limit since the start of a potential disordered breathing event, a rate of oxygen desaturation of successive oxygen saturation measurements exceeding a predetermined desaturation limit, or encountering an invalid oxygen saturation measurement, wherein the successive oxygen saturation measurements are temporally after the second oxygen saturation measurement. In various embodiments of the method, validating the potential disordered breathing event includes evaluating all of the conditions to determine whether any one of the conditions is satisfied. In various embodiments of the method, the predetermined increase duration is one second.

In various embodiments of the method, determining the disordered breathing events includes determining a duration of the potential disordered breathing event between the start and the end of the potential disordered breathing event, determining a magnitude of oxygen desaturation over the duration of the potential disordered breathing event, and recording the potential disordered breathing events as one of the disordered breathing events. In various embodiments of the method, the method includes recording the potential disordered breathing event as one of the disordered breathing events when the duration of the potential disordered breathing event is equal to or exceeds a predetermined minimum duration, and the magnitude of oxygen desaturation over the duration is equal to or exceeds a predetermined minimum oxygen desaturation magnitude.

In various embodiments of the method, for a recorded disordered breathing event, the method includes recording the duration of and the magnitude of oxygen desaturation of the recorded disordered breathing event.

In various embodiments of the method, determining the disordered breathing events includes identifying a potential disordered breathing event by a duration during which SO₂ oxygen saturation measurements in the oxygen saturation data are below a predetermined SO₂ event threshold, and determining that the potential disordered breathing event is not a disordered breathing event by at least one condition. The conditions include at least one of: the duration is shorter than a predetermined minimum duration, a magnitude of SO₂ desaturation over the duration is less than a predetermined minimum SO₂ desaturation threshold, or the potential disordered breathing event includes an initial SO₂ desaturation rate that is equal to or exceeds a predetermined maximum SO₂ desaturation rate.

In various embodiments of the method, determining the disordered breathing events further includes disregarding any SO₂ oxygen saturation measurements that are below a predetermined minimum SO₂ threshold.

In various embodiments of the method, the potential disordered breathing event ends when successive SO₂ oxygen saturation measurements are unchanged for a predetermined maximum plateau duration.

In various embodiments of the method, the method includes recording the potential disordered breathing event as one of the disordered breathing events, and categorizing the potential disordered breathing event into a category. The categories may include a significant drop event where the magnitude of SO₂ desaturation over the duration is equal to or exceeds a predetermined significant drop threshold, a below-critical event where any SO₂ oxygen saturation measurements are below a predetermined critically-low value, and/or a significant drop and below-critical event where the magnitude of SO₂ desaturation over the duration is equal to or exceeds the predetermined significant drop threshold and any SO₂ oxygen saturation measurements are below the predetermined critically-low value.

In various embodiments of the method, the method includes accessing recorded cyclic variation of heart rate data obtained from the person, combining the cardiopulmonary coupling data and the cyclic variation of heart rate data to provide CPC-CVHR data, wherein the CPC-CVHR data is correlated in time with the oxygen saturation data over the time period, and determining a type of CPC-CVHR event for each of the disordered breathing events based on the CPC-CVHR data corresponding in time to the disordered breathing events.

In various embodiments of the method, determining the type of CPC-CVHR event for each of the disordered breathing events includes selecting, for each of the disordered breathing events, one CPC-CVHR event type from the group of: high frequency coupling without CVHR (HFC), HFC with CVHR (HFC_{CVHR}), low frequency coupling without CVHR (LFC), LFC with CVHR (LFC_{CVHR}), very low frequency coupling without CVHR (vLFC), vLFC with CVHR (vLFC_{CVHR}), elevated low frequency coupling broad band without CVHR (eLFCBB), eLFCBB with CVHR (eLFCBB_{CVHR}), elevated low frequency coupling narrow band without CVHR (eLFCNB), and eLFCNB with CVHR (eLFCNB_{CVHR}).

In various embodiments of the method, determining the type of CPC-CVHR event for each of the disordered breathing events includes selecting, for each of the disordered breathing events, one CPC-CVHR event type from the group of: high frequency coupling without CVHR (HFC), HFC with CVHR (HFC_{CVHR}), low frequency coupling without CVHR (LFC), LFC with CVHR (LFC_{CVHR}), REM without CVHR (REM), REM with CVHR (REM_{CVHR}), wake without CVHR (WAKE), wake with CVHR (WAKE_{CVHR}), elevated low frequency coupling broad band without CVHR (eLFCBB), eLFCBB with CVHR (eLFCBB_{CVHR}), elevated low frequency coupling narrow band without CVHR (eLFCNB), and eLFCNB with CVHR (eLFCNB_{CVHR}).

In various embodiments of the method, the method includes determining a total number of events for each of the event types including: HFC, HFC with CVHR, LFC, LFC with CVHR, REM, REM with CVHR, wake, wake with CVHR, eLFCBB, eLFCBB with CVHR, eLFCNB, and eLFCNB with CVHR.

In various embodiments of the method, the method includes determining a sleep disorder prevalence measure based on total number of the disordered breathing events, determining a sleep disorder severity measure based on a distribution of the disordered breathing events across the event types, the distribution being based on the total number of events for each of the event types, and determining a measure of sleep apnea based on the sleep disorder prevalence measure and the sleep disorder severity measure.

In various embodiments of the method, the method includes determining, for each of the event types, an average magnitude of desaturation for events in the event type and an average rate of desaturation for events in the event type, accessing a magnitude threshold and a rate threshold, and determining whether the person experienced hypopnea or apnea based on comparing the average magnitude of desaturation for each event type with the magnitude threshold, and comparing the average rate of desaturation for each event type with the rate threshold.

In various embodiments of the method, determining whether the person experienced hypopnea or apnea is based further on at least one of a total duration of the disordered breathing events, an average oxygen saturation for the disordered breathing events and a distribution of the disordered breathing events across the event types.

In accordance with aspects of the present disclosure, a system of sleep assessment includes one or more processors, and at least one memory storing instructions which, when executed by the one or more processors, cause the system to access oxygen saturation data including oxygen saturation measurements for a person over a time period, determine disordered breathing events for the person over the time period based on the oxygen saturation data, access cardiopulmonary coupling data for the person during sleep wherein the cardiopulmonary coupling data is correlated in time with the oxygen saturation data over the time period, and determine a type of cardiopulmonary coupling event for each of the disordered breathing events which were determined based on the oxygen saturation data based on the cardiopulmonary coupling data corresponding in time to the disordered breathing events which were determined based on the oxygen saturation data.

In various embodiments of the system, the instructions, when executed by the processor, further cause the system to determine, based on the oxygen saturation data, that the disordered breathing events are sleep disordered breathing events.

In various embodiments of the system, in determining the disordered breathing events, the instructions, when executed by the processor, cause the system to identify a start of a potential disordered breathing event by a decrease between a first oxygen saturation measurement and a second oxygen saturation measurement in the oxygen saturation data, wherein the second oxygen saturation measurement is temporally after the first oxygen saturation measurement, and validating the potential disordered breathing event by at least one condition.

In various embodiments of the system, the condition includes at least one of successive oxygen saturation measurements remaining unchanged for a predetermined plateau duration, successive oxygen saturation measurements increasing for a predetermined increase duration, reaching a predetermined duration limit since the start of a potential disordered breathing event, a rate of oxygen desaturation of successive oxygen saturation measurements exceeding a predetermined desaturation limit, or encountering an invalid oxygen saturation measurement, wherein the successive oxygen saturation measurements are temporally after the second oxygen saturation measurement. In various embodiments of the system, in validating the potential disordered breathing event, the instructions, when executed by the processor, cause the system to evaluate all of the conditions to determine whether any one of the conditions is satisfied. In various embodiments of the system, the predetermined increase duration is one second.

In various embodiments of the system, in determining the disordered breathing events, the instructions, when executed by the processor, cause the system to determine a duration of the potential disordered breathing event between the start and the end of the potential disordered breathing event, determine a magnitude of oxygen desaturation over the duration of the potential disordered breathing event, and record the potential disordered breathing event as one of the disordered breathing events. In various embodiments of the system, the system records the potential disordered breathing event as one of the disordered breathing events when the duration of the potential disordered breathing event is equal to or exceeds a predetermined minimum duration, and the magnitude of oxygen desaturation over the duration is equal to or exceeds a predetermined minimum oxygen desaturation.

In various embodiments of the system, the instructions, when executed by the processor, further cause the system to, for a recorded disordered breathing event, record the duration of and the magnitude of oxygen desaturation of the recorded disordered breathing event.

In various embodiments of the system, in determining the disordered breathing events, the instructions, when executed by the processor, cause the system to identify a potential disordered breathing event by a duration during which SO₂ oxygen saturation measurements in the oxygen saturation data are below a predetermined SO₂ event threshold, and determine that the potential disordered breathing event is not a disordered breathing event by at least one condition. The at least one condition includes one or more of: the duration is shorter than a predetermined minimum duration, a magnitude of SO₂ desaturation over the duration is less than a predetermined minimum SO₂ desaturation threshold, or the potential disordered breathing event includes an initial SO₂ desaturation rate that is equal to or exceeds a predetermined maximum SO₂ desaturation rate.

In various embodiments of the system, in determining the disordered breathing events, the instructions, when executed by the processor, cause the system to disregard any SO₂ oxygen saturation measurements that are below a predetermined minimum SO₂ threshold.

In various embodiments of the system, the potential disordered breathing event ends when successive SO₂ oxygen saturation measurements are unchanged for a predetermined plateau duration.

In various embodiments of the system, the instructions, when executed by the processor, further cause the system to record the potential disordered breathing event as one of the disordered breathing events, and categorize the potential disordered breathing event into a category. The categories include a significant drop event where the magnitude of SO₂ desaturation over the duration is equal to or exceeds a predetermined significant drop threshold, a below-critical event where any SO₂ oxygen saturation measurements are below a predetermined critically-low value, and a significant drop and below-critical event where the magnitude of SO₂ desaturation over the duration is equal to or exceeds the predetermined significant drop threshold and any SO₂ oxygen saturation measurements are below the predetermined critically-low value.

In various embodiments of the system, the instructions, when executed by the processor, further cause the system to access cyclic variation of heart rate data for the person during apnea, combine the cardiopulmonary coupling data and the cyclic variation of heart rate data to provide CPC-CVHR data, wherein the CPC-CVHR data is correlated in time with the oxygen saturation data over the time period, and determine a type of CPC-CVHR event for each of the disordered breathing events based on the CPC-CVHR data corresponding in time to the disordered breathing events.

In various embodiments of the system, in determining the type of CPC-CVHR event for each of the disordered breathing events, the instructions, when executed by the processor, cause the system to select, for each of the disordered breathing events, one CPC-CVHR event type from the group of: high frequency coupling without CVHR (HFC), HFC with CVHR (HFC_{CVHR}), low frequency coupling without CVHR (LFC), LFC with CVHR (LFC_{CVHR}), very low frequency coupling without CVHR (vLFC), vLFC with CVHR (vLFC_{CVHR}), elevated low frequency coupling broad band without CVHR (eLFCBB), eLFCBB with CVHR (eLFCBB_{CVHR}), elevated low frequency coupling narrow band without CVHR (eLFCNB), and eLFCNB with CVHR (eLFCNB_{CVHR}).

In various embodiments of the system, in determining the type of CPC-CVHR event for each of the disordered breathing events, the instructions, when executed by the processor, cause the system to select, for each of the disordered breathing events, one CPC-CVHR event type from the group of: high frequency coupling without CVHR (HFC), HFC with CVHR (HFC_{CVHR}), low frequency coupling without CVHR (LFC), LFC with CVHR (LFC_{CVHR}), REM without CVHR (REM), REM with CVHR (REM_{CVHR}), wake without CVHR (WAKE), wake with CVHR (WAKE_{CVHR}), elevated low frequency coupling broad band without CVHR (eLFCBB), eLFCBB with CVHR (eLFCBB_{CVHR}), elevated low frequency coupling narrow band without CVHR (eLFCNB), and eLFCNB with CVHR (eLFCNB_{CVHR}).

In various embodiments of the system, the instructions, when executed by the processor, further cause the system to determine a total number of events for each of the event types including: HFC, HFC with CVHR, LFC, LFC with CVHR, REM, REM with CVHR, wake, wake with CVHR, eLFCBB, eLFCBB with CVHR, eLFCNB, and eLFCNB with CVHR.

In various embodiments of the system, the instructions, when executed by the processor, further cause the system to determine a sleep disorder prevalence measure based on total duration of the disordered breathing events, determine a sleep disorder severity measure based on a distribution of the disordered breathing events across the event types where the distribution is based on the total number of events for each of the event types, and determine a measure of sleep apnea based on the sleep disorder prevalence measure and the sleep disorder severity measure.

In various embodiments of the system, the instructions, when executed by the processor, further cause the system to determine, for each of the event types, an average magnitude of desaturation for events in the event type and an average rate of desaturation for events in the event type, access a magnitude threshold and a rate threshold, determine whether the person experienced hypopnea or apnea based on comparing the average magnitude of desaturation for each event type with the magnitude threshold, and compare the average rate of desaturation for each event type with the rate threshold.

In various embodiments of the system, determining whether the person experienced hypopnea or apnea is based further on at least one of a total duration of the disordered breathing events, an average oxygen saturation for the disordered breathing events, and a distribution of the disordered breathing events across the event types.

### BRIEF DESCRIPTION OF THE DRAWINGS

Objects and features of the presently disclosed system and method will become apparent to those of ordinary skill in the art when descriptions of various embodiments thereof are read with reference to the accompanying drawings, wherein:
FIG. 1 is an illustration of certain aspects of the present disclosure;
FIG. 2 is a flowchart of an exemplary method for analysis of sleep disordered breathing events using oxygen saturation and cardiopulmonary coupling data, provided in accordance with aspects of the present disclosure;
FIG. 3 is a flowchart of an exemplary method for determining a potential disordered breathing event, provided in accordance with aspects of the present disclosure;
FIG. 4 is a flowchart of an exemplary method for determining and recording a disordered breathing event, provided in accordance with aspects of the present disclosure;
FIG. 5 is a flowchart of another exemplary method for determining and recording a potential disordered breathing event, provided in accordance with an embodiment of the present disclosure;
FIG. 6 is a flowchart of an exemplary method for analysis of sleep disordered breathing events by combining oxygen saturation and cardiopulmonary coupling analysis with analysis of cyclic variation of heart rate, provided in accordance with aspects of the present disclosure;
FIG. 7 is an illustration of an example of identifying a potential sleep disordered event based on oxygen saturation data and analysis, provided in accordance with aspects of the present disclosure;
FIG. 8 is an illustration of an example of an adult study with predominantly central apnea, provided in accordance with aspects of the present disclosure; and
FIG. 9 is a diagram of an exemplary system in accordance with aspects of the present disclosure.

### DETAILED DESCRIPTION

The present disclosure relates to analyzing sleep disordered breathing events. In accordance with aspects of the present disclosure, the analysis is based on oxygen saturation data and cardiopulmonary coupling data. In various embodiments, the analysis is further based on cyclic variation of heart rate data. Using a combination of such data provides more sophisticated analyses than traditional analyses such as apnea-hypopnea index, which simply reflects the number of sleep apnea or hypopnea events per hour of sleep.

Cardiopulmonary coupling is described in, among others, U.S. Patent No. 7,324,845, U.S. Patent No. 7,734,334, U.S. Patent No. 8,403,848, and U.S. Patent No. 8,401,626. Cardiopulmonary coupling is a technology for assessing sleep quality by performing a quantitative analysis between two physiological signals - an N-N interval series from Heart Rate Variability coupled with corresponding direct or derived respiration signals -- to determine the coherent cross-power of these two signals. The coherent cross-power in turn provides a measurement of cardiopulmonary coupling that can be used to differentiate obstructive from non-obstructive sleep disordered breathing disease. In various embodiments, the two physiological signals can be derived from various physiological measures.

In detecting sleep disordered breathing utilizing cardiopulmonary coupling, the types of CPC events include high frequency coupling, low frequency coupling, and very low frequency coupling, among others which will be described in more detail later herein. High frequency coupling represents stable sleep which is a biomarker of integrated stable NREM sleep and is associated with periods of stable breathing, high vagal tone, generally a non-cyclic alternating pattern on the electroencephalogram (EEG), high relative delta power, physiologic blood pressure dipping (in health), and/or stable arousal threshold. Low frequency coupling represents unstable sleep which is a biomarker of integrated unstable NREM sleep, with opposite features to Stable sleep. Unstable sleep is associated with EEG activities called cyclic alternating pattern (CAP), periods of fluctuating breathing patterns (tidal volume fluctuations), cyclic variation of heart rate (CVHR), blood pressure non-dipping, and/or variable arousal thresholds. Fragmented REM sleep has low-frequency coupling characteristics. Very low frequency coupling represents REM sleep and wake. Other CPC events will be described in more detail later herein. The proportion of time that a person's sleep is characterized by the various CPC events can be used to assess sleep disorders, including sleep breathing disorders.

Oxygen saturation is a measure of the degree to which hemoglobin contained in the red blood cells has bonded with oxygen molecules. Oxygen saturation can be quantified in different ways, and the terms "oxygen saturation" or "SO₂" are used herein as generic descriptor for SaO₂ and SpO₂, which correspond to different ways of collecting oxygen saturation data. In various embodiments, oxygen saturation data includes blood oxygen saturation measurements indicating the percentage of hemoglobin molecules in the arterial blood which are saturated with oxygen.

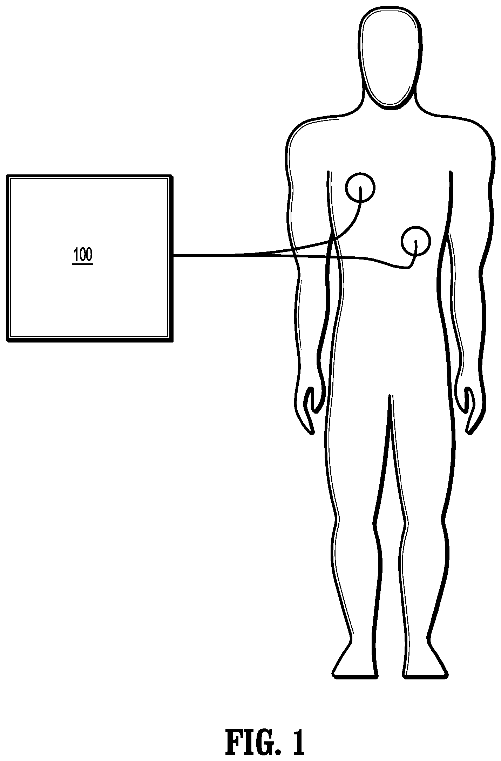

Referring now to FIG. 1, there is shown an illustration of a system 100 in accordance with aspects of the present disclosure. The system 100 can be attached to a person during sleep to obtain physiological measurements that can be used to compute cardiopulmonary coupling ("CPC"), such as electrocardiogram measurements or other physiological measurements. The system 100 also obtains oxygen saturation measurements. The various sensors for detecting the physiological signals and the oxygen saturation will be understood by persons skilled in the art. The physiological measurements can be recorded in a storage medium, such as a disk drive, flash drive, solid state drive, or other storage medium. In various embodiments, the physiological measurements for computing CPC and the oxygen saturation measurements can be recorded in parallel. In various embodiments, each recorded data can be tagged or associated with a time stamp. In various embodiments, the physiological measurements can be used to determine the CPC events over time, and the CPC events can be recorded in the storage medium. In various embodiments, the CPC events over time can be tagged or associated with a time stamp. By tagging or associating recorded data with time stamps, various recorded measurements can be correlated in time. The disclosed embodiments are exemplary, and it is contemplated that other ways of correlating recorded measurements in time can be employed.

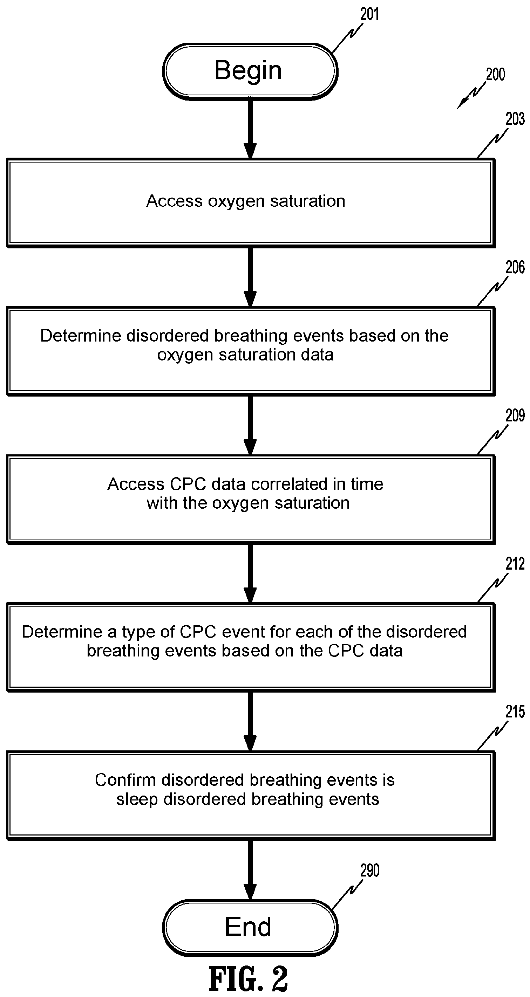

FIG. 2 shows a flowchart of an operation for analyzing sleep disordered breathing events using oxygen saturation and cardiopulmonary coupling data. The assessment of sleep disordered breathing events can use oxygen saturation data and cardiopulmonary coupling data recorded in the storage medium discussed in connection with FIG. 1. In summary, oxygen saturation data can be used to identify potential sleep disordered breathing events, and CPC data can be used to categorize the potential disordered breathing events. In various embodiments, the disclosed operation can be implemented by software instructions executing on one or more processors.

At step 203, oxygen saturation (SO₂) data is accessed. As explained above, SO₂ is used herein as a generic descriptor for oxygen saturation and may include SaO₂ and/or SpO₂ for different data collection methods. In various embodiments, the oxygen saturation data includes blood oxygen saturation readings indicating the percentage of hemoglobin molecules in the arterial blood which are saturated with oxygen. At step 206, a disordered breathing event is determined based on the accessed oxygen saturation data. In various embodiments, the potential disordered breathing event may be determined by detecting periods of declining SO₂ or periods where SO₂ is below a certain threshold. In various embodiments, the SO₂ analysis output can be used to confirm whether the event caused a drop in SO₂ as a result of sleep apnea, as opposed to an arousal caused by a sleep disorder other than sleep disordered breathing.

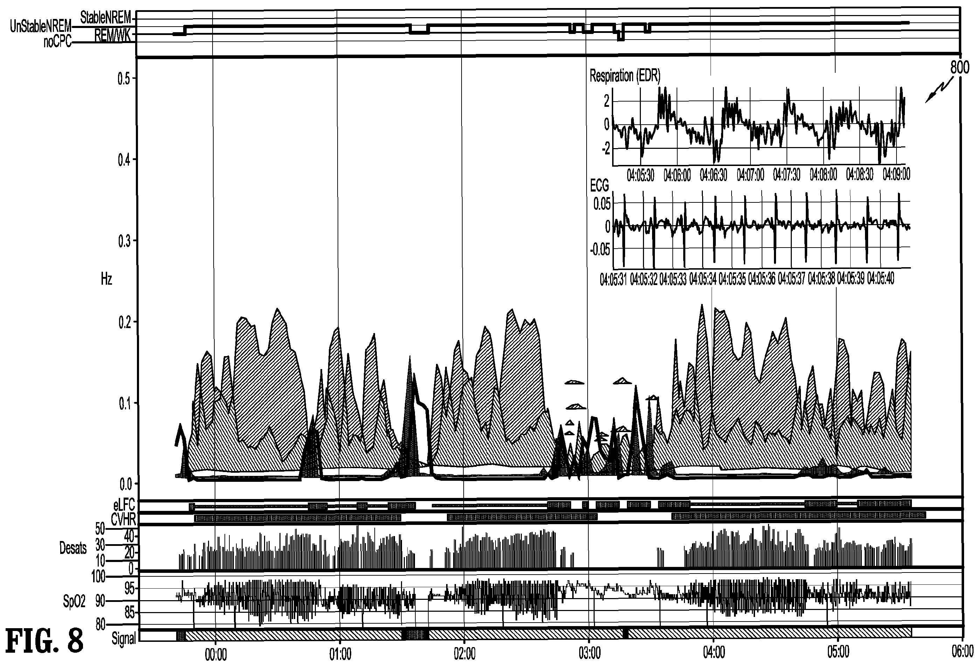

At step 209, cardiopulmonary coupling (CPC) data correlated in time with the oxygen saturation data is accessed. CPC data may be based on coupling heart rate variability (HRV) and respiration to generate frequency maps of coupled autonomic-respiratory oscillations, such as the frequency map shown in FIG. 8. In various embodiments, the CPC frequency analysis of the coupling between heart rate variability (HRV) and respiration during sleep can include at least three frequency bands, including High Frequency, Low Frequency and Very Low Frequency. The high frequency band includes frequencies greater than 0.1 Hz, the low frequency band includes frequencies between 0.01 Hz to 0.1 Hz, inclusive, and the very low frequency range includes frequencies less than 0.01 Hz.

As mentioned above, high frequency represents stable sleep which is a biomarker of integrated stable NREM sleep. Low frequency represents unstable sleep which is a biomarker of integrated unstable NREM sleep, with opposite features to stable sleep. Fragmented REM sleep has low-frequency coupling characteristics. Very low frequency represents REM sleep and awake state. Low frequency coupling can be further sub-categorized as elevated low frequency coupling broad band (eLFC_{BB}) or fragmentation, elevated low frequency coupling narrow band (eLFC_{NB}) or periodicity, or no elevated low frequency coupling. Additionally, each type of CPC can include cyclic variation of heart rate (CVHR) or no CVHR. Each of these types of cardiopulmonary coupling will be referred to herein as a "CPC event."

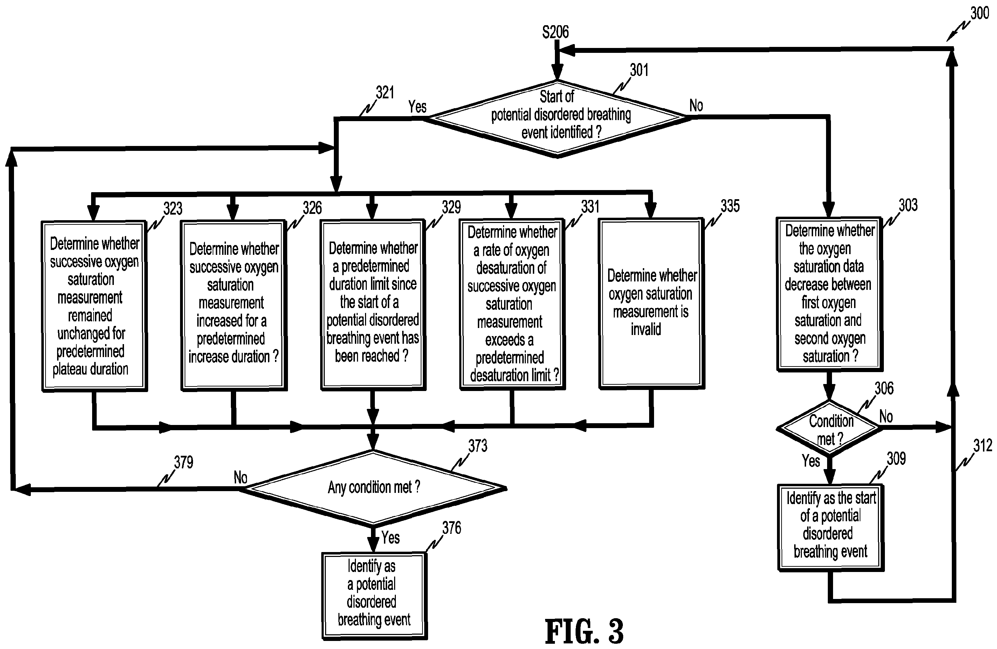

At step 212, a type of CPC event is determined for each of the disordered breathing events based on the CPC data. In various embodiments, the CPC events can include high frequency coupling without CVHR (HFC), HFC with CVHR (HFC_{CVHR}), low frequency coupling without CVHR (LFC), LFC with CVHR (LFC_{CVHR}), very low frequency coupling without CVHR (vLFC), very low frequency coupling with CVHR (vLFC_{CVHR}), elevated low frequency coupling broad band without CVHR (eLFCBB), elevated low frequency coupling broad band with CVHR (eLFCBB_{CVHR}), elevated low frequency coupling narrow band without CVHR (eLFCNB), and elevated low frequency coupling narrow band with CVHR (eLFCNB_{CVHR}). The CPC events are utilized to identify the type and characteristic of sleep disorders, which will be described in more detail later herein. In the illustrated embodiment, step 215 confirms whether the disordered breathing events are sleep disordered breathing events. Afterwards, the sleep assessment operation ends. Various portions of the operation of FIG. 2 will now be described in more detail in connection with FIGS. 3-5.

Referring to FIG. 3, there is shown a method for determining a potential disordered breathing event. In various embodiments, FIG. 3 occurs within step 206 of FIG. 2 and involves identifying a start of a potential disordered breathing event and validating the potential disordered breathing event. At step 301, oxygen saturation data is used to determine whether the start of a potential disordered breathing event has already been identified. If not, at step 303, the start of a potential disordered breathing event can be identified based on the oxygen saturation decreasing between a first oxygen saturation measurement and a second subsequent oxygen saturation measurement. That is, a decrease between successive oxygen saturation measurements can be identified as a start of a potential disordered breathing event. If there has been a decrease, as determined at step 306, *i.e.,* if the oxygen saturation data decreased between the first oxygen saturation measurement and the second subsequent oxygen saturation measurement, then at step 309, the decreased oxygen saturation is identified as the start of the potential disordered breathing event. But if there has been no decrease in oxygen saturation, as determined at step 306, the oxygen saturation data is analyzed over time until a decrease is detected.

At step 312, once the start of the potential disordered breathing event has been identified, the oxygen saturation data is further analyzed to determine whether a potential disordered breathing event is validated, based on a set of conditions 323, 326, 329, 331, and 335. Condition 323 identifies whether successive oxygen saturation measurements have remained unchanged for a predetermined plateau duration. Condition 326 identifies whether successive oxygen saturation measurements have increased for a predetermined increase duration. In some embodiments, the predetermined increase duration is one second. In various embodiments, the predetermined increase duration can be another time period. Condition 329 identifies whether a predetermined duration limit since the start of the potential disordered breathing event has been reached. Condition 331 identifies whether a rate of oxygen desaturation of successive oxygen saturation measurement exceeds a predetermined desaturation limit. Condition 335 identifies whether the oxygen saturation measurement is invalid. For example, an oxygen saturation measurement may be invalid due to data corruption in collection, in the storage medium, or other factors. Step 373 determines whether any of the conditions is satisfied. At step 376, if at least one of the set of conditions 323, 326, 329, 331, or 335, is met, then the potential disordered breathing event is validated and recorded. At step 379, if none of the set of conditions 323, 326, 329, 331, or 335, is met, the oxygen saturation data is further analyzed until one of the conditions 323, 326, 329, 331, or 335, is met. Accordingly, by the operation of FIG. 3, the start and validity of a potential disordered breathing event can be identified based on oxygen saturation data. A disordered breathing event ends when the oxygen saturation increases. The disclosed embodiments are exemplary, and variations are contemplated to be within the scope of the present disclosure. For example, conditions other than the conditions disclosed above can be used to identify a start of or to validate a potential disordered breathing event.

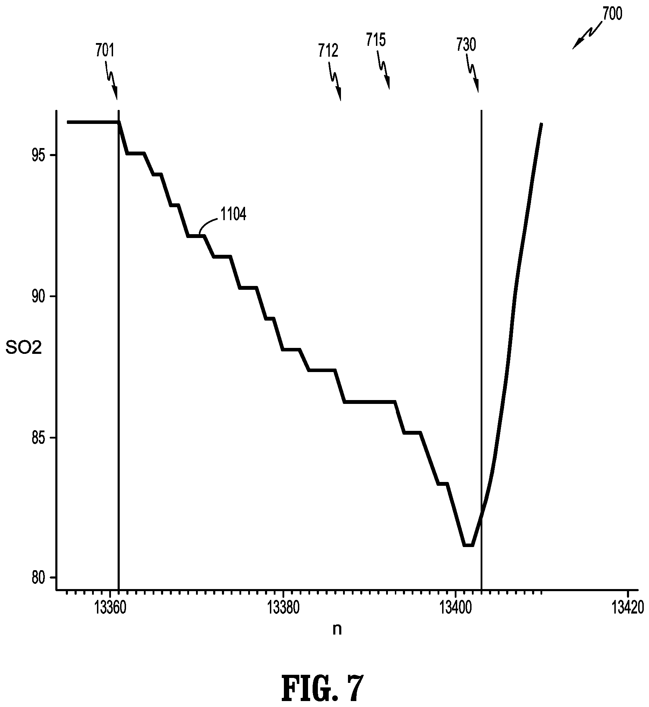

FIG. 7 shows an illustration of an example of identifying a potential disordered breathing event based on oxygen saturation data and analysis. With continuing reference to FIG. 3, at step 303 of FIG 3, the start of the potential disordered breathing is determined based on the oxygen saturation data decreasing between a first oxygen saturation measurement and a second subsequent oxygen saturation measurement. At step 309, because the oxygen saturation data decreased between the first oxygen saturation measurement and the second subsequent oxygen saturation measurement, the decreased oxygen saturation measurement is identified as the start of a potential disordered breathing event. FIG. 7 shows that the SO₂ data experiences a drop, which marks a start of a potential event 701. Once the drop is detected and the start of a potential disordered breathing event is identified, at step 312 of FIG 3, the oxygen saturation data is analyzed until at least one of the set of conditions 323, 326, 329, 331, or 335, is met, at which point the potential disordered breathing event 730 is validated and recorded. With respect to condition 323, which identifies whether the successive oxygen saturation measurements remained unchanged for a predetermined plateau duration, FIG. 7 shows an example of a plateau occurring between measurements 712 and 715 followed by additional desaturation. In various embodiments, if this plateau satisfies condition 323, such a plateau would be identified as the end of the potential disordered breathing. In the example of FIG. 7, the plateau does not satisfy condition 323 and the analysis of the oxygen saturation measurements continues until measurement 730. Measurement 730 could satisfy, for example, condition 326 or condition 329, such that the potential disordered breathing event is validated. The example of FIG. 7 is exemplary and does not limit the scope of the present disclosure.

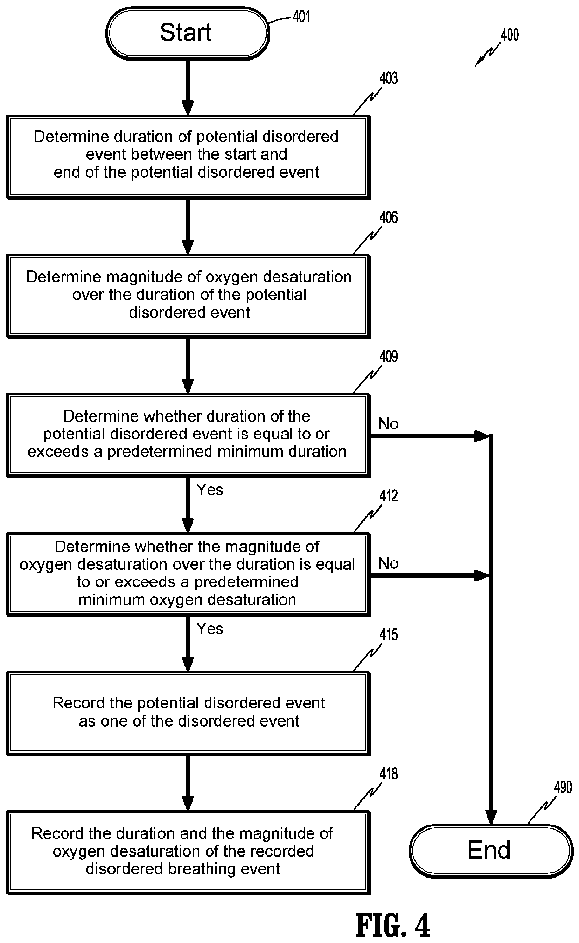

Referring now to FIG. 4, a method 400 is disclosed for validating and recording, or not recording, disordered breathing events that were identified in the operation of FIG. 3. In various embodiments, the operation of FIG. 4 can also occur in step 206 of FIG. 2. Once both the start and the end of a potential disordered breathing event are identified, then at step 403, a duration of the potential disordered breathing event between the start and the end of the potential disordered breathing event is evaluated. As mentioned above, a potential disordered breathing event ends when successive oxygen saturation measurements increase. At step 406, a magnitude of oxygen desaturation over the duration of the potential disordered breathing event is evaluated. Based on the duration determined at step 403, the duration of the potential disordered breathing is compared to a predetermined minimum duration at step 409, to determine if the duration of the potential disordered breathing event is equal to or exceeds the predetermined minimum duration. At step 412, the magnitude of oxygen desaturation is compared to a predetermined minimum oxygen desaturation, to determine if the magnitude of oxygen desaturation is equal to or exceeds the predetermined minimum oxygen desaturation. At step 418, if the duration of the potential disordered breathing event is equal or exceeds the predetermined minimum duration and the magnitude of oxygen desaturation is equal or exceeds the predetermined minimum oxygen desaturation, then the potential disordered breathing event is validated and is recorded as a disordered breathing event. However, if the duration of the potential disordered breathing event less than the predetermined minimum duration or the magnitude of oxygen desaturation is less than the predetermined minimum oxygen desaturation, then the potential disordered event is not validated and is not recorded. In various embodiments, at step 418, the duration and the magnitude of oxygen desaturation of the recorded disordered breathing event are also recorded with the recorded disordered breathing event. In various embodiments, the SO₂ analysis does not require a predetermined baseline value to validate a disordered breathing event.

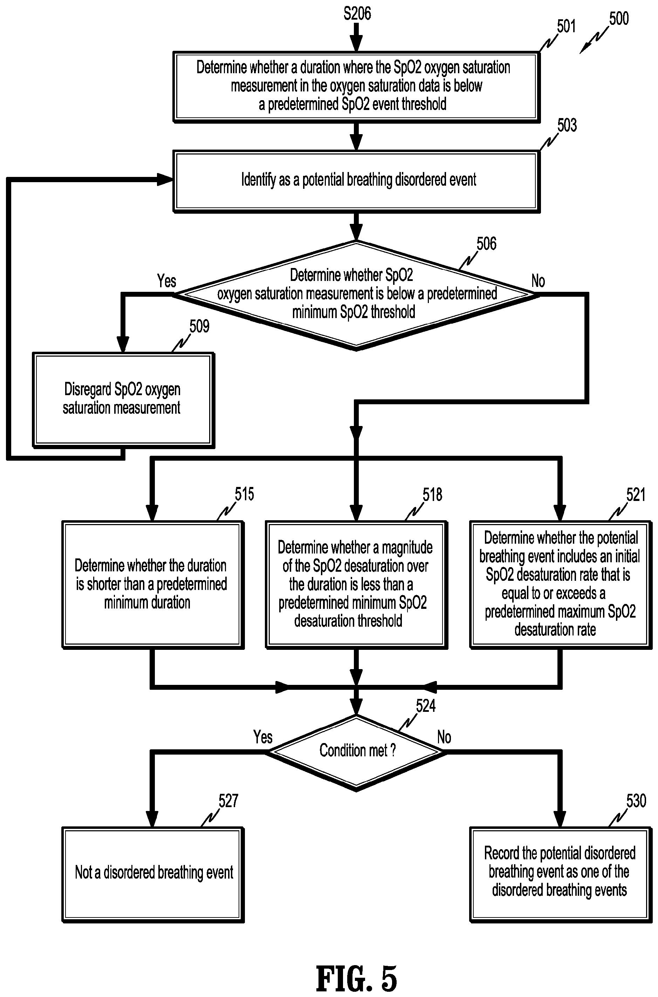

Referring to FIG. 5, another method is disclosed for validating and recording, or not recording, disordered breathing events that were identified in the operation of FIG. 3. In various embodiments, the operation of FIG. 5 can also occur in step 206 of FIG. 2. In various embodiments, the operation of FIG. 5 can occur before or after the operation of FIG. 4. The illustrated operation of FIG. 5 is based on SpO₂ oxygen saturation, but it will be understood that the operation is applicable to other oxygen saturation measurements as well. At step 501, a minimum SpO₂ threshold value is accessed for the SpO₂ oxygen saturation measurements in the oxygen saturation data. At step 503, a potential disordered breathing event is identified based on, for example, the operation of FIG. 3. In steps 506 and 509, the operation removes certain oxygen saturation measurements. Step 506 determines whether a SpO₂ oxygen saturation measurement is below the predetermined minimum SpO₂ threshold. At step 509, if the SpO₂ oxygen saturation measurement is below the predetermined minimum SpO₂ threshold, the SpO₂ oxygen saturation measurement is disregarded. The potential disordered breathing event is formed by the remaining oxygen saturation measurements, and the potential disordered breathing event is analyzed to validate and record, or not record, the potential disordered breathing event based on various conditions. At step 515, the operation evaluates whether the duration of the potential oxygen desaturation event is shorter than a predetermined minimum duration. At step 518, the operation evaluates whether the magnitude of SpO₂ desaturation over the duration is less than a predetermined minimum SpO₂ desaturation threshold. At step 521, the operation evaluates whether the potential disordered breathing event includes an initial SpO₂ desaturation rate that is equal to or exceeds a predetermined maximum SpO₂ desaturation rate. In various embodiments, the evaluation of step 521 can be based on the oxygen saturation measurements that are used to identify the start of the potential disordered breathing event. At step 524, the operation evaluates if any of the conditions 515, 518, or 521 are met. If any of the conditions are met, then at step 527, the potential disordered breathing event is not considered a sleep disordered breathing event and is not recorded. At step 530, if none of the conditions are met, the potential disordered breathing event is recorded as a sleep disordered breathing event.

In accordance with aspects of the present disclosure, the recorded disordered breathing event can be categorized by determining whether it exhibits a significant drop event where the magnitude of SpO₂ desaturation over the duration is equal to or exceeds a predetermined significant drop threshold, a below-critical event where any of the SpO₂ oxygen saturation measurements are below a predetermined critically-low value, and/or a significant drop and below critical event where the magnitude of SpO₂ desaturation over the duration is equal to or exceeds the predetermined significant drop threshold and any of the SpO₂ oxygen saturation measurements are below the predetermined critically-low value. In various embodiments, each category parameter may have a default value, which can be determined during calibration but can be changed based on specific requirements. In some embodiments, each disordered breathing event can be characterized with a start date, an event duration, a desaturation percentage drop, a desaturation rate of drop, and/or lowest SO₂ measurement data.

Accordingly, described above herein are exemplary operations for identifying potential disordered breathing events and for validating and recording, or not recording, the potential disordered breathing events. The following will describe categorizing the recorded disordered breathing events based on cardiopulmonary coupling ("CPC") and Cyclic Variation of Heart Rate ("CVHR") data and performing sleep assessment.

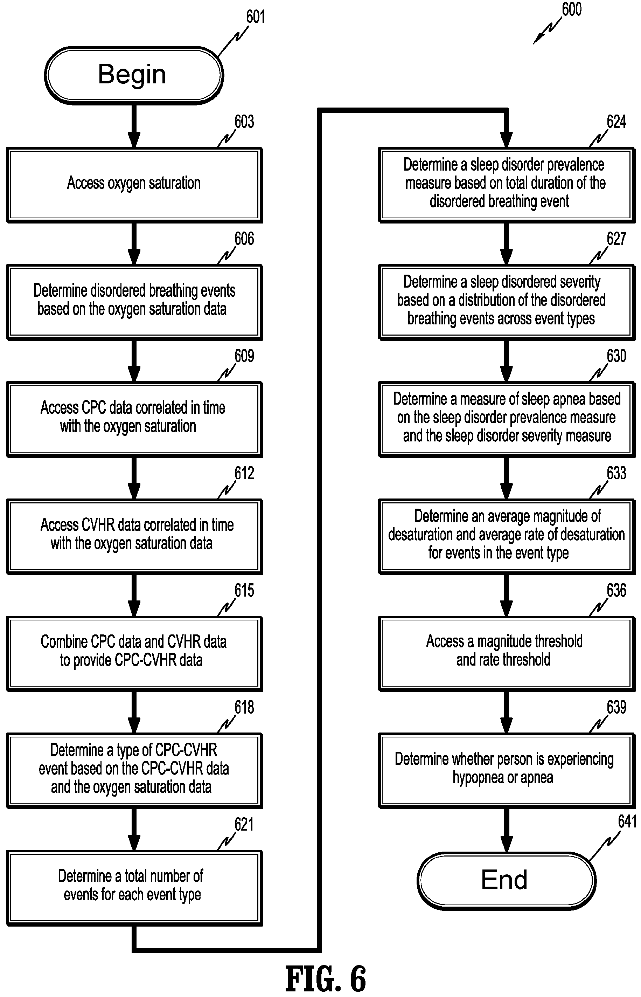

In accordance with aspects of the present disclosure, and with reference to FIG. 6, there is shown a method of analysis of sleep disordered breathing events based on cardiopulmonary coupling analysis and based on cyclic variation of heart rate characteristics. Steps 603 and 606 can be implemented by the operations of FIGS. 3-5 described above herein. At step 603, oxygen saturation (SO₂) data is accessed. At step 606, a disordered breathing event is determined based on the accessed oxygen saturation data. The traditional Apnea-Hypopnea Index is presented as events per hour and is interpreted as a severity index by comparing the events per hour to defined thresholds, with higher prevalence of apnea and hypopnea events indicating more severe conditions. However, the information provided by this traditional index is limited. In accordance with aspects of the present disclosure, CPC and CVHR data are used to categorize the disordered breathing events to provide further information on a subject's condition. CPC reflects the coupling of cardiovascular and pulmonary mechanisms, and the CPC events can be correlated to the subject's state, which is determined by the autonomic nervous system. Therefore, the result of the events can define the severity of a subject's condition.

Cardiopulmonary coupling (CPC) data correlated in time with the oxygen saturation data is accessed, at step 609. At step 612, cyclic variations in heart rate (CVHR) data correlated in time with the oxygen saturation data is accessed at step 612. Based on the CPC and the CVHR data, step 615 combines the two to provide CPC-CVHR data. At step 618, a type of CPC-CVHR event is determined for each of the sleep disordered breathing events based on the CPC-CVHR data. As described above herein, in various embodiments, the events can include high frequency coupling without CVHR (HFC), HFC with CVHR (HFC_{CVHR}), low frequency coupling without CVHR (LFC), LFC with CVHR (LFC_{CVHR}), very low frequency coupling without CVHR (vLFC), very low frequency coupling with CVHR (vLFC_{CVHR}), elevated low frequency coupling broad band without CVHR (eLFCBB), elevated low frequency coupling broad band with CVHR (eLFCBB_{CVHR}), elevated low frequency coupling narrow band without CVHR (eLFCNB), and elevated low frequency coupling narrow band with CVHR (eLFCNB_{CVHR}).

In accordance with aspects of the present disclosure, the CPC-CVHR events may be used in evaluating the sleep state of a person and in phenotyping the events. For example, eLFCBB is a marker of fragmentation and is associated with obstructive apneas (OA). eLFCNB is a marker of periodicity and is associated with periodic breathing (PB), Cheyne-Stokes respiration (CS), and central apneas (CA). However, eLFCBB can be caused by other disorders such as pain or other disturbances during sleep that cause fragmentation, while eLFCNB can be caused by periodic limb movements. As another example, a large number of events in "eLFCNB + CVHR" is more severe than the same number of events in "HFC". This is because the eLFCNB + CVHR events simultaneously reflect periodicity and the cardiovascular signature of bradycardia followed by tachycardia (CVHR).

In accordance with aspects of the present disclosure, the total count/duration of events is used as a measure of prevalence of sleep disorder, while the categories where the events occur define the severity of the sleep disorder. Additionally, the average event duration, average desaturation, and average desaturation rate can be used to define the severity within a category.

In various embodiments, determining a type of CPC-CVHR event for each of the disordered breathing events may include selecting, for each of the disordered breathing events, one CPC-CVHR event type from high frequency coupling without CVHR (HFC), HFC with CVHR (HFC_{CVHR}), low frequency coupling without CVHR (LFC), LFC with CVHR (LFC_{CVHR}), very low frequency coupling without CVHR (vLFC), vLFC with CVHR (vLFC_{CVHR}), elevated low frequency coupling broad band without CVHR (eLFCBB), eLFCBB with CVHR (eLFCBB_{CVHR}), elevated low frequency coupling narrow band without CVHR (eLFCNB), and eLFCNB with CVHR (eLFCNB_{CVHR}).

In various embodiments, determining a type of CPC-CVHR event for each of the disordered breathing events includes selecting, for each of the disordered breathing events, one of CPC-CVHR event type from high frequency coupling without CVHR (HFC), HFC with CVHR (HFC_{CVHR}), low frequency coupling without CVHR (LFC), LFC with CVHR (LFC_{CVHR}), REM without CVHR (REM), REM with CVHR (REM_{CVHR}), wake without CVHR (WAKE), wake with CVHR (WAKE_{CVHR}), elevated low frequency coupling broad band without CVHR (eLFCBB), eLFCBB with CVHR (eLFCBB_{CVHR}), elevated low frequency coupling narrow band without CVHR (eLFCNB), and eLFCNB with CVHR (eLFCNB_{CVHR}). At step 621, once the type of CPC-CVHR event for each of the disordered breathing events is determined, a total number of events for each of the event types is determined. The event types that are counted may include HFC without CVHR, HFC with CVHR, LFC without CVHR, LFC with CVHR, REM without CVHR, REM with CVHR, wake without CVHR, wake with CVHR, eLFCBB without CVHR, eLFCBB with CVHR, eLFCNB without CVHR, and eLFCNB with CVHR.

At step 624, a sleep disorder prevalence is measured based on the total duration of the disordered breathing event. At step 627, a sleep disorder severity is measured based on the distribution of the disordered breathing events across the event types, based on the total number of events for each of the event types. At step 630, a measure of sleep apnea is measured based on the measured sleep disorder breathing prevalence and the sleep disorder severity. For example, with reference to FIG. 8, in a 352-minute adult study with predominantly central apnea, the person was determined to have an AHI of 67.3 (events/hour), with 178 minutes in apnea, and with 134 of those 178 minutes occurring in eLFCNB.

In accordance with aspects of the present disclosure, the events in each category are further analyzed. At step 633, for each of the event types, the operation determines an average magnitude of desaturation and the maximum desaturation among events in the event type, as well as an average, maximum, and minimum rate of desaturation among the events in the event type. Additionally, in various embodiments, the operation can determine for each event type an average, maximum, and minimum duration among the events. Then at step 636, a magnitude threshold and a rate threshold are accessed, and at step 639, the operation evaluates whether the person experienced hypopnea or apnea based on the various metrics and thresholds. For example, comparing the average magnitude of desaturation for each event type with the magnitude threshold can be used in determining whether the subject experienced hypopnea or apnea. In various embodiments, determining whether the person experienced hypopnea or apnea may be further based on at least one of a total duration of the disordered breathing events, an average oxygen saturation for the disordered breathing events, and a distribution of disordered breathing events across the event types.

In accordance with aspects of the present disclosure, based on comparing thresholds to the desaturation percentage and the desaturation rate in each event category, and identifying the CPC categories, hypopneas and apneas can be distinguished. Hypopneas are normally described as abnormally shallow breathing while apneas are defined as a cessation of breathing. As a result of the difference in severity of these two respiratory event types, the oxygen saturation response is different in severity and is characterized by different desaturation low (DL), magnitude (DM), rate (DR), and acceleration (DA). For example, in various embodiments, the values of DM, DR, and/or DA exceeding fixed or variable threshold values are indicative of apneas while values below the threshold are indicative of hypopneas. Events may then be summarized as an Apnea-Hypopnea Index (including all events), Apnea Index (apneas only), and Hypopnea Index (hypopneas only). Additionally, events can be listed with their apnea/hypopnea classifications and the associated severity characteristics (DL, DM, DR, and DA).

By analyzing the characteristics of the events and the distribution of events between categories, the present disclosure generates a measure for the severity of the disorder that is more informative than the traditional legacy AHI. In accordance with aspects of the present disclosure, prevalence (number of events per hour), and severity (DM and DR), category concentration (in which state most of the apneas occur), and the average oxygen saturation, can be combined to generate a number that is indicative of the severity of the disorder. The number is referred to herein as sAHI. Additionally, in various embodiments, a traditional legacy AHI is generated, which is calculated as (Total Number of Events) / (Total Sleep Time).

The following provides an example of severity by category type:

| | Subject A with CVHR | without CVHR | | Subject B with CVHR | without CVHR |
|---|---|---|---|---|---|
| HFC | *6* | 45 | HFC | *0* | 0 |
| LFC | *0* | 26 | LFC | *20* | 0 |
| vLFC | *0* | 22 | vLFC | *2* | 0 |
| eLFCBB | **3** | 3 | eLFCBB | **28** | *16* |
| eLFCNB | **0** | *0* | eLFCNB | **30** | *9* |
| Total | 9 | 96 | Total | 80 | 25 |

Subject A experienced events that mostly concentrated in non-CVHR and non-eLFC categories (normal font). Subject B on the other hand had events concentrated in CVHR and eLFC categories, which indicates that the events resulted in a cardiovascular response in addition to occurring in eLFCNB-a state associated with central sleep apnea and periodic breathing. Both subjects experienced the same number of events and therefore have the same prevalence. Assuming a 7-hour sleep duration, both subject A and B would have 15 events per hour, based on dividing the number of events by the sleep duration. By weighing the events by the CPC-CVHR types in which they occur, the severity indices are calculated for each subject, where the resulting metric more accurately reflects severity by incorporating CPC-CVHR, which is indicative of autonomic nervous system activity in response to sleep disordered breathing events. For example, a severity measure could be generated by assigning weights to each cell in the table with HFC-without-CVHR having the lowest weight and eLFCNB-with-CVHR having the highest weight. Each cell is represented as a ratio of events in that category over total events, which is then multiplied by the weight for the associated category, divided by the maximum weight. The resulting values are then added, generating a number in [0, 1]. As an example, if events only occurred in HFC-without-CVHR, the severity metric would be equal to zero (0). If events only occurred in eLFCNB-with-CVHR, the severity metric would be one (1). This can be represented with the following equation, where severity (S) is within [0, 1]: $S = {\sum }_{i = 1}^{5} {\sum }_{j = 2}^{2} \frac{{x}_{i , j} {y}_{i , j}}{T ∗ MAX \left(Y\right)} ,$ where T is the total number of events, x_{i,j} and y_{i,j} are elements in matrices X (event counts) and Y (category weights), respectively, and MAX(Y) is the maximum of Y.

FIGS. 2-6 are exemplary illustrations that explain embodiments of the present disclosure. It should be understood that embodiments of the present disclosure could be implemented in hardware, firmware, software, or a combination thereof. In such an embodiment, the various components and steps would be implemented in hardware, firmware, and/or software to perform the functions of the present disclosure. That is, the same piece of hardware, firmware, or module of software could perform one or more of the illustrated blocks (i.e., components or steps).

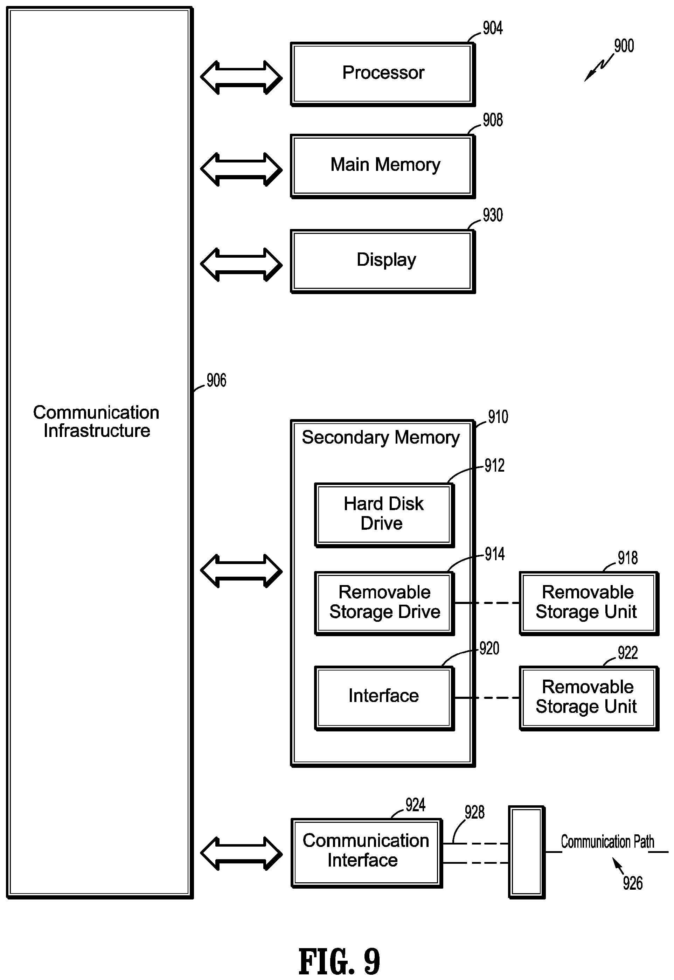

The present disclosure can be implemented in one or more computer systems capable of carrying out the functionality described herein. Referring to FIG. 9, an example of a computer system 900 for implementing the present disclosure is shown. Various embodiments of the disclosure described herein can be implemented by the computer system 900. However, it will be apparent to one skilled in the relevant art(s) how to implement the disclosure using other computer systems and/or computer architectures.

The computer system 900 includes one or more processors, such as processor 904. The processor 904 is connected to a communication infrastructure 906 (e.g., a communications bus, crossover bar, or network).

Computer system 900 can include a display 930 that receives graphics, text, and other data from the communication infrastructure 906 (or from a frame buffer not shown) for display. In various embodiments, the display 930 can present various measurements and metrics described herein, including the oxygen saturation and the sAHI score described above herein. In various embodiments, the sAHI score can be displayed with oxygen saturation (SO2) to aid clinical decisions for sleep disordered breathing (SDB). In various embodiments, the display 930 can present graphical and numerical presentations and reports of sleep latency, sleep duration, sleep quality, and/or sleep pathology for the use by or on the order of physicians, trained technicians, or other healthcare professionals, among others. The presentations and reports can include some or all of the various metrics disclosed above herein.

Computer system 900 also includes a main memory 908, preferably random access memory (RAM), and can also include a secondary memory 910. The secondary memory 910 can include, for example, a hard disk drive 912 and/or a removable storage drive 914, representing a floppy disk drive, a magnetic tape drive, an optical disk drive, etc. The removable storage drive 914 reads from and/or writes to a removable storage unit 918 in a well-known manner. Removable storage unit 918, represents a floppy disk, magnetic tape, optical disk, etc. which is read by and written to removable storage drive 914. As will be appreciated, the removable storage 918 includes a computer usable storage medium having stored therein computer software (e.g., programs or other instructions) and/or data.

In various embodiments, secondary memory 910 can include other similar devices for allowing computer software and/or data to be loaded into computer system 900. Such devices can include, for example, a removable storage 922 and an interface 920. Examples of such can include a program cartridge and cartridge interface (such as that found in legacy devices), a removable memory chip (such as an EPROM, or PROM) and associated socket, and other removable storage devices 922 and interfaces 920 which allow software and data to be transferred from the removable storage device 922 to computer system 900.

Computer system 900 can also include a communications interface 924. Communications interface 924 allows software and data to be transferred between computer system 900 and external devices. Examples of communications interface 924 can include a modem, a network interface (such as an Ethernet or WiFi card), a communications port, a PCMCIA or SD or other slot and card, among other components. Software and data transferred via communications interface 924 are in the form of signals 928 which can be electronic, electromagnetic, optical, or other signals capable of being received by communications interface 924. These signals 928 are provided to communications interface 924 via a communications path (i.e., channel) 926. Communications path 926 carries signals 928 and can be implemented using wire or cable, fiber optics, a phone line, a cellular phone link, an RF link, free-space optics, and/or other communications channels.

As used herein, the terms "computer program medium" and "computer usable medium" are used to generally refer to media such as removable storage 918, removable storage 922, a hard disk installed in hard disk drive 912, and signals 928. These computer program products are devices for providing software to computer system 900. The present disclosure includes such computer program products.

Computer programs (also called computer control logic or computer readable program code) are stored in main memory 908 and/or secondary memory 910. Computer programs can also be received via communications interface 924. Such computer programs, when executed, enable the computer system 900 to implement the present disclosure as discussed herein. In particular, the computer programs, when executed, enable the processor 904 to implement the processes and operations of the present disclosure, such as the various steps of methods 200, 300, 400, 500, and 600, for example, described above. Accordingly, such computer programs represent controllers of the computer system 900.

In an embodiment where the disclosure is implemented using software, the software can be stored in a computer program product and loaded into computer system 900 using removable storage drive 914, hard drive 912, interface 920, or communications interface 924. The control logic (software), when executed by the processor 904, causes the processor 904 to perform the functions of the disclosure as described herein. Accordingly, the technology of the present disclosure may be provided as software as a medical device (SaMD) or as a non-medical software. In various embodiments, the software may include cloud-based applications.

The embodiments disclosed herein are examples of the disclosure and may be embodied in various forms. For instance, although certain embodiments herein are described as separate embodiments, each of the embodiments herein may be combined with one or more of the other embodiments herein. Specific structural and functional details disclosed herein are not to be interpreted as limiting, but as a representative basis for teaching one skilled in the art to variously employ the present disclosure in virtually any appropriately detailed structure.

The phrases "in an embodiment," "in embodiments," "in various embodiments," "in some embodiments," or "in other embodiments" may each refer to one or more of the same or different embodiments in accordance with the present disclosure. A phrase in the form "A or B" means "(A), (B), or (A and B)." A phrase in the form "at least one of A, B, or C" means "(A); (B); (C); (A and B); (A and C); (B and C); or (A, B, and C)."

Any of the herein described methods, programs, algorithms or codes may be converted to, or expressed in, a programming language or computer program. The terms "programming language" and "computer program," as used herein, each include any language used to specify instructions to a computer, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, Python, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, a program, where the programming language could exist in more than one state (such as source, compiled, object, or linked) is a reference to any and all such states.

The systems described herein may also utilize one or more controllers to receive various information and transform the received information to generate an output. The controller may include any type of computing device, computational circuit, or any type of processor or processing circuit capable of executing a series of instructions that are stored in a memory. The controller may include multiple processors and/or multicore central processing units (CPUs) and may include any type of processor, such as a microprocessor, digital signal processor, microcontroller, programmable logic device (PLD), field programmable gate array (FPGA), or the like. The controller may also include a memory to store data and/or instructions that, when executed by the one or more processors, causes the one or more processors to perform one or more methods and/or algorithms.

It should be understood that the foregoing description is only illustrative of the present disclosure. The embodiments described with reference to the attached drawing figures are presented only to demonstrate certain examples of the disclosure. The invention is defined by the claims.

## Claims

1. A method of sleep assessment, comprising:
accessing recorded oxygen saturation data (203) including oxygen saturation measurements for a person over a time period;
determining disordered breathing events (206) for the person over the time period based on the oxygen saturation data;
accessing recorded cardiopulmonary coupling data (209) obtained from the person during sleep, wherein the cardiopulmonary coupling data is correlated in time with the oxygen saturation data over the time period; and
determining a type of cardiopulmonary coupling event (212) for each of the disordered breathing events which were determined based on the oxygen saturation data, based on the cardiopulmonary coupling data corresponding in time to the disordered breathing events which were determined based on the oxygen saturation data.

2. The method according to claim 1, wherein determining the disordered breathing events (206) includes:
identifying a start of a potential disordered breathing event (301) by a decrease between a first oxygen saturation measurement and a second oxygen saturation measurement in the oxygen saturation data, wherein the second oxygen saturation measurement is temporally after the first oxygen saturation measurement; and
validating the potential disordered breathing event (321) by at least one of conditions including:
successive oxygen saturation measurements remaining unchanged for a predetermined plateau duration (323),
successive oxygen saturation measurements increasing for a predetermined increase duration (326),
reaching a predetermined duration limit since the start of a potential disordered breathing event (329),
a rate of oxygen desaturation of successive oxygen saturation measurements exceeding a predetermined desaturation limit (331), or
encountering an invalid oxygen saturation measurement (335),
wherein the successive oxygen saturation measurements are temporally after the second oxygen saturation measurement.

3. The method according to claim 1, wherein determining the disordered breathing events includes:
identifying a potential disordered breathing event by a duration during which SO₂ oxygen saturation measurements in the oxygen saturation data are below a predetermined SO₂ event threshold (506); and
determining that the potential disordered breathing event is not a disordered breathing event by at least one of conditions including:
the duration is shorter than a predetermined minimum duration (515),
a magnitude of SO₂ desaturation over the duration is less than a predetermined minimum SO₂ desaturation threshold (518), or
the potential disordered breathing event includes an initial SO₂ desaturation rate that is equal to or exceeds a predetermined maximum SO₂ desaturation rate (521).

4. The method according to claim 1, further comprising,
accessing recorded cyclic variation of heart rate data (612) obtained from the person;
combining the cardiopulmonary coupling data and the cyclic variation of heart rate data (615) to provide CPC-CVHR data, wherein the CPC-CVHR data is correlated in time with the oxygen saturation data over the time period; and
determining a type of CPC-CVHR event (618) for each of the disordered breathing events based on the CPC-CVHR data corresponding in time to the disordered breathing events.

5. The method according to claim 4, wherein determining the type of CPC-CVHR event (618) for each of the disordered breathing events includes selecting, for each of the disordered breathing events, one CPC-CVHR event type from the group of: high frequency coupling without CVHR (HFC), HFC with CVHR (HFC_{CVHR}), low frequency coupling without CVHR (LFC), LFC with CVHR (LFC_{CVHR}), REM without CVHR (REM), REM with CVHR (REM_{CVHR}), wake without CVHR (WAKE), wake with CVHR (WAKE_{CVHR}), elevated low frequency coupling broad band without CVHR (eLFCBB), eLFCBB with CVHR (eLFCBB_{CVHR}), elevated low frequency coupling narrow band without CVHR (eLFCNB), and eLFCNB with CVHR (eLFCNB_{CVHR}).

6. The method according to claim 5, further comprising determining a total number of events (621) for each of the event types including: HFC, HFC with CVHR, LFC, LFC with CVHR, REM, REM with CVHR, wake, wake with CVHR, eLFCBB, eLFCBB with CVHR, eLFCNB, and eLFCNB with CVHR.

7. The method according to claim 6, further comprising:
determining a sleep disorder prevalence measure based on total number of the disordered breathing events;
determining a sleep disorder severity measure (627) based on a distribution of the disordered breathing events across the event types, the distribution being based on the total number of events for each of the event types; and
determining a measure of sleep apnea (630) based on the sleep disorder prevalence measure and the sleep disorder severity measure.

8. The method according to claim 6, further comprising:
determining, for each of the event types, an average magnitude of desaturation (633) for events in the event type and an average rate of desaturation for events in the event type;
accessing a magnitude threshold and a rate threshold (636); and
determining whether the person experienced hypopnea or apnea (639) based on comparing the average magnitude of desaturation for each event type with the magnitude threshold, and
comparing the average rate of desaturation for each event type with the rate threshold.

9. The method according to claim 8, wherein determining whether the person experienced hypopnea or apnea is based further on at least one of a total duration of the disordered breathing events, an average oxygen saturation for the disordered breathing events, and a distribution of the disordered breathing events across the event types.

10. A system (900) for sleep assessment, comprising:
one or more processors (904); and
at least one memory (908, 910) storing instructions which, when executed by the one or more processors, cause the system to:
access oxygen saturation data including oxygen saturation measurements for a person over a time period;
determine disordered breathing events for the person over the time period based on the oxygen saturation data;
access cardiopulmonary coupling data for the person during sleep, wherein the cardiopulmonary coupling data is correlated in time with the oxygen saturation data over the time period; and
determine a type of cardiopulmonary coupling event for each of the disordered breathing events which were determined based on the oxygen saturation data, based on the cardiopulmonary coupling data corresponding in time to the disordered breathing events which were determined based on the oxygen saturation data.

11. The system according to claim 10, wherein in determining the disordered breathing events, the instructions, when executed by the processor, cause the system to:
identify a start of a potential disordered breathing event by a decrease between a first oxygen saturation measurement and a second oxygen saturation measurement in the oxygen saturation data, wherein the second oxygen saturation measurement is temporally after the first oxygen saturation measurement; and
validating the potential disordered breathing event by at least one of conditions including:
successive oxygen saturation measurements remaining unchanged for a predetermined plateau duration,
successive oxygen saturation measurements increasing for a predetermined increase duration,
reaching a predetermined duration limit since the start of a potential disordered breathing event,
a rate of oxygen desaturation of successive oxygen saturation measurements exceeding a predetermined desaturation limit, or
encountering an invalid oxygen saturation measurement,
wherein the successive oxygen saturation measurements are temporally after the second oxygen saturation measurement.

12. The system according to claim 10, wherein in determining the disordered breathing events, the instructions, when executed by the processor, cause the system to:
identify a potential disordered breathing event by a duration during which SO₂ oxygen saturation measurements in the oxygen saturation data are below a predetermined SO₂ event threshold; and
determine that the potential disordered breathing event is not a disordered breathing event by at least one of conditions including:
the duration is shorter than a predetermined minimum duration,
a magnitude of SO₂ desaturation over the duration is less than a predetermined minimum SO₂ desaturation threshold, or
the potential disordered breathing event includes an initial SO₂ desaturation rate that is equal to or exceeds a predetermined maximum SO₂ desaturation rate.

13. The system according to claim 10, wherein the instructions, when executed by the processor, further cause the system to:
access cyclic variation of heart rate data for the person;
combine the cardiopulmonary coupling data and the cyclic variation of heart rate data to provide CPC-CVHR data, wherein the CPC-CVHR data is correlated in time with the oxygen saturation data over the time period; and
determine a type of CPC-CVHR event for each of the disordered breathing events based on the CPC-CVHR data corresponding in time to the disordered breathing events.

14. The system according to claim 13, wherein in determining the type of CPC-CVHR event for each of the disordered breathing events, the instructions, when executed by the processor, cause the system to select, for each of the disordered breathing events, one CPC-CVHR event type from the group of: high frequency coupling without CVHR (HFC), HFC with CVHR (HFC_{CVHR}), low frequency coupling without CVHR (LFC), LFC with CVHR (LFC_{CVHR}), REM without CVHR (REM), REM with CVHR (REM_{CVHR}), wake without CVHR (WAKE), wake with CVHR (WAKE_{CVHR}), elevated low frequency coupling broad band without CVHR (eLFCBB), eLFCBB with CVHR (eLFCBB_{CVHR}), elevated low frequency coupling narrow band without CVHR (eLFCNB), and eLFCNB with CVHR (eLFCNB_{CVHR}).

15. The system according to claim 14, wherein the instructions, when executed by the processor, further cause the system to determine a total number of events for each of the event types including: HFC, HFC with CVHR, LFC, LFC with CVHR, REM, REM with CVHR, wake, wake with CVHR, eLFCBB, eLFCBB with CVHR, eLFCNB, and eLFCNB with CVHR.

16. The system according to claim 15, wherein the instructions, when executed by the processor, further cause the system to:
determine a sleep disorder prevalence measure based on total number of the disordered breathing events;
determine a sleep disorder severity measure based on a distribution of the disordered breathing events across the event types, the distribution being based on the total number of events for each of the event types; and
determine a measure of sleep apnea based on the sleep disorder prevalence measure and the sleep disorder severity measure.

17. The system according to claim 15, wherein the instructions, when executed by the processor, further cause the system to:
determine, for each of the event types, an average magnitude of desaturation for events in the event type and an average rate of desaturation for events in the event type;
access a magnitude threshold and a rate threshold; and
determine whether the person experienced hypopnea or apnea based on comparing the average magnitude of desaturation for each event type with the magnitude threshold, and
compare the average rate of desaturation for each event type with the rate threshold.

18. The system according to claim 17, wherein determining whether the person experienced hypopnea or apnea is based further on at least one of a total duration of the disordered breathing events, an average oxygen saturation for the disordered breathing events, and a distribution of the disordered breathing events across the event types.

## Patentansprüche

1. Verfahren zur Schlafbeurteilung, umfassend:
Zugreifen auf aufgezeichnete Sauerstoffsättigungsdaten (203), einschließend Sauerstoffsättigungsmessungen, für eine Person über einen Zeitraum;
Bestimmen von Atmungsstörungsereignissen (206) für die Person über den Zeitraum auf der Basis der Sauerstoffsättigungsdaten;
Zugreifen auf aufgezeichnete kardiopulmonale Kopplungsdaten (209), die von der Person während des Schlafes bezogen werden, wobei die kardiopulmonalen Kopplungsdaten mit den Sauerstoffsättigungsdaten über den Zeitraum zeitlich korreliert werden; und
Bestimmen eines Typs eines kardiopulmonalen Kopplungsereignisses (212) für jedes der Atmungsstörungsereignisse, die auf der Basis der Sauerstoffsättigungsdaten bestimmt wurden, auf der Basis der kardiopulmonalen Kopplungsdaten, die den Atmungsstörungsereignissen zeitlich entsprechen, die auf der Basis der Sauerstoffsättigungsdaten bestimmt wurden.

2. Verfahren nach Anspruch 1, wobei das Bestimmen der Atmungsstörungsereignisse (206) einschließt:
Identifizieren eines Starts eines potenziellen Atmungsstörungsereignisses (301) durch eine Abnahme zwischen einer ersten Sauerstoffsättigungsmessung und einer zweiten Sauerstoffsättigungsmessung in den Sauerstoffsättigungsdaten, wobei die zweite Sauerstoffsättigungsmessung zeitlich nach der ersten Sauerstoffsättigungsmessung ist; und
Validieren des potenziellen Atmungsstörungsereignisses (321) durch mindestens eine von Bedingungen, einschließend:
sukzessive Sauerstoffsättigungsmessungen bleiben für eine vorbestimmte Plateaudauer unverändert (323),
sukzessive Sauerstoffsättigungsmessungen nehmen für eine vorbestimmte Zunahmedauer zu (326),
Erreichen einer vorbestimmten Dauergrenze seit dem Start eines potenziellen Atmungsstörungsereignisses (329),
eine Sauerstoffentsättigungsrate von sukzessiven Sauerstoffsättigungsmessungen übersteigt eine vorbestimmte Entsättigungsgrenze (331), oder
Stoßen auf eine ungültige Sauerstoffsättigungsmessung (335),
wobei die sukzessiven Sauerstoffsättigungsmessungen zeitlich nach der zweiten Sauerstoffsättigungsmessung sind.

3. Verfahren nach Anspruch 1, wobei das Bestimmen der Atmungsstörungsereignisse einschließt:
Identifizieren eines potenziellen Atmungsstörungsereignisses durch eine Dauer, während der SO₂-Sauerstoffsättigungsmessungen in den Sauerstoffsättigungsdaten unter einem vorbestimmten SO₂-Ereignisschwellenwert liegen (506); und
Bestimmen, dass das potenzielle Atmungsstörungsereignis kein Atmungsstörungsereignis ist, durch mindestens eine von Bedingungen, einschließend:
die Dauer ist kürzer als eine vorbestimmte minimale Dauer (515),
eine Größenordnung von SO₂-Entsättigung über die Dauer ist kleiner als ein vorbestimmter minimaler SO₂-Entsättigungsschwellenwert (518), oder
das potenzielle Atmungsstörungsereignis schließt eine anfängliche SO₂-Entsättigungsrate ein, die gleich einer vorbestimmten maximalen SO₂-Entsättigungsrate ist oder diese übersteigt (521).

4. Verfahren nach Anspruch 1, ferner umfassend,
Zugreifen auf eine aufgezeichnete zyklische Variation von Herzfrequenzdaten (612), die von der Person bezogen werden;
Kombinieren der kardiopulmonalen Kopplungsdaten und der zyklischen Variation von Herzfrequenzdaten (615), um CPC-CVHR-Daten bereitzustellen, wobei die CPC-CVHR-Daten mit den Sauerstoffsättigungsdaten über den Zeitraum zeitlich korreliert werden; und
Bestimmen eines Typs eines CPC-CVHR-Ereignisses (618) für jedes der Atmungsstörungsereignisse auf der Basis der CPC-CVHR-Daten, die den Atmungsstörungsereignissen zeitlich entsprechen.

5. Verfahren nach Anspruch 4, wobei das Bestimmen des Typs eines CPC-CVHR-Ereignisses (618) für jedes der Atmungsstörungsereignisse ein Auswählen eines CPC-CVHR-Ereignistyps für jedes der Atmungsstörungsereignisse aus der Gruppe einschließt: hochfrequente Kopplung ohne CVHR (HFC), HFC mit CVHR (HFC_{CVHR}), niederfrequente Kopplung ohne CVHR (LFC), LFC mit CVHR (LFC_{CVHR}), REM ohne CVHR (REM), REM mit CVHR (REM_{CVHR}), Aufwachen ohne CVHR (WAKE), Aufwachen mit CVHR (WAKE_{CVHR}), erhöhtes breites Frequenzband mit niederfrequenter Kopplung ohne CVHR (eLFCBB), eLFCBB mit CVHR (eLFCBB_{CVHR}), erhöhtes schmales Frequenzband mit niederfrequenter Kopplung ohne CVHR (eLFCNB) und eLFCNB mit CVHR (eLFCNB_{CVHR}).

6. Verfahren nach Anspruch 5, ferner umfassend ein Bestimmen einer Gesamtanzahl von Ereignissen (621) für jeden der Ereignistypen, einschließend: HFC, HFC mit CVHR, LFC, LFC mit CVHR, REM, REM mit CVHR, Aufwachen, Aufwachen mit CVHR, eLFCBB, eLFCBB mit CVHR, eLFCNB und eLFCNB mit CVHR.

7. Verfahren nach Anspruch 6, ferner umfassend:
Bestimmen eines Schlafstörungsprävalenzmaßes auf der Basis der Gesamtanzahl der Atmungsstörungsereignisse;
Bestimmen eines Schlafstörungsschweregradmaßes (627) auf der Basis einer Verteilung der Atmungsstörungsereignisse über die Ereignistypen, wobei die Verteilung auf der Gesamtanzahl von Ereignissen für jeden der Ereignistypen basiert ist; und
Bestimmen eines Maßes von Schlafapnoe (630) auf der Basis des Schlafstörungsprävalenzmaßes und des Schlafstörungsschweregradmaßes.

8. Verfahren nach Anspruch 6, ferner umfassend:
Bestimmen einer durchschnittlichen Entsättigungsgrößenordnung (633) für jeden der Ereignistypen für Ereignisse in dem Ereignistyp und einer durchschnittlichen Entsättigungsrate für Ereignisse in dem Ereignistyp;
Zugreifen auf einen Größenordnungsschwellenwert und einen Ratenschwellenwert (636); und
Bestimmen, ob die Person eine Hypopnoe oder eine Apnoe erfahren hat (639), auf der Basis eines Vergleichens der durchschnittlichen Entsättigungsgrößenordnung für jeden Ereignistyp mit dem Größenordnungsschwellenwert, und
Vergleichen der durchschnittlichen Entsättigungsrate für jeden Ereignistyp mit dem Ratenschwellenwert.

9. Verfahren nach Anspruch 8, wobei das Bestimmen, ob die Person eine Hypopnoe oder eine Apnoe erfahren hat, ferner auf mindestens einer von einer Gesamtdauer der Atmungsstörungsereignisse, einer durchschnittlichen Sauerstoffsättigung für die Atmungsstörungsereignisse und einer Verteilung der Atmungsstörungsereignisse über die Ereignistypen basiert.

10. System (900) zur Schlafbeurteilung, umfassend:
einen oder mehrere Prozessoren (904); und
mindestens einen Speicher (908, 910), der Anweisungen speichert, die bei Ausführung durch den einen oder die mehreren Prozessoren das System veranlassen zum:
Zugreifen auf Sauerstoffsättigungsdaten, einschließend Sauerstoffsättigungsmessungen, für eine Person über einen Zeitraum;
Bestimmen von Atmungsstörungsereignissen für die Person über den Zeitraum auf der Basis der Sauerstoffsättigungsdaten;
Zugreifen auf kardiopulmonale Kopplungsdaten für die Person während des Schlafes, wobei die kardiopulmonalen Kopplungsdaten mit den Sauerstoffsättigungsdaten über den Zeitraum zeitlich korreliert werden; und
Bestimmen eines Typs eines kardiopulmonalen Kopplungsereignisses für jedes der Atmungsstörungsereignisse, die auf der Basis der Sauerstoffsättigungsdaten bestimmt wurden, auf der Basis der kardiopulmonalen Kopplungsdaten, die den Atmungsstörungsereignissen zeitlich entsprechen, die auf der Basis der Sauerstoffsättigungsdaten bestimmt wurden.

11. System nach Anspruch 10, wobei beim Bestimmen der Atmungsstörungsereignisse die Anweisungen bei Ausführung durch den Prozessor das System veranlassen zum:
Identifizieren eines Starts eines potenziellen Atmungsstörungsereignisses durch eine Abnahme zwischen einer ersten Sauerstoffsättigungsmessung und einer zweiten Sauerstoffsättigungsmessung in den Sauerstoffsättigungsdaten, wobei die zweite Sauerstoffsättigungsmessung zeitlich nach der ersten Sauerstoffsättigungsmessung ist; und
Validieren des potenziellen Atmungsstörungsereignisses durch mindestens eine von Bedingungen, einschließend:
sukzessive Sauerstoffsättigungsmessungen bleiben für eine vorbestimmte Plateaudauer unverändert,
sukzessive Sauerstoffsättigungsmessungen nehmen für eine vorbestimmte Zunahmedauer zu,
Erreichen einer vorbestimmten Dauergrenze seit dem Start eines potenziellen Atmungsstörungsereignisses,
eine Sauerstoffentsättigungsrate von sukzessiven Sauerstoffsättigungsmessungen übersteigt eine vorbestimmte Entsättigungsgrenze, oder
Stoßen auf eine ungültige Sauerstoffsättigungsmessung,
wobei die sukzessiven Sauerstoffsättigungsmessungen zeitlich nach der zweiten Sauerstoffsättigungsmessung sind.

12. System nach Anspruch 10, wobei beim Bestimmen der Atmungsstörungsereignisse die Anweisungen bei Ausführung durch den Prozessor das System veranlassen zum:
Identifizieren eines potenziellen Atmungsstörungsereignisses durch eine Dauer, während der SO₂-Sauerstoffsättigungsmessungen in den Sauerstoffsättigungsdaten unter einem vorbestimmten SO₂-Ereignisschwellenwert liegen; und
Bestimmen, dass das potenzielle Atmungsstörungsereignis kein Atmungsstörungsereignis ist, durch mindestens eine von Bedingungen, einschließend:
die Dauer ist kürzer als eine vorbestimmte minimale Dauer,
eine Größenordnung von SO₂-Entsättigung über die Dauer ist kleiner als ein vorbestimmter minimaler SO₂-Entsättigungsschwellenwert, oder
das potenzielle Atmungsstörungsereignis schließt eine anfängliche SO₂-Entsättigungsrate ein, die gleich einer vorbestimmten maximalen SO₂-Entsättigungsrate ist oder diese übersteigt.

13. System nach Anspruch 10, wobei die Anweisungen bei Ausführung durch den Prozessor das System ferner veranlassen zum:
Zugreifen auf eine zyklische Variation von Herzfrequenzdaten für die Person;
Kombinieren der kardiopulmonalen Kopplungsdaten und der zyklischen Variation von Herzfrequenzdaten, um CPC-CVHR-Daten bereitzustellen, wobei die CPC-CVHR-Daten mit den Sauerstoffsättigungsdaten über den Zeitraum zeitlich korreliert werden; und
Bestimmen eines Typs eines CPC-CVHR-Ereignisses für jedes der Atmungsstörungsereignisse auf der Basis der CPC-CVHR-Daten, die den Atmungsstörungsereignissen zeitlich entsprechen.

14. System nach Anspruch 13, wobei beim Bestimmen des Typs eines CPC-CVHR-Ereignisses für jedes der Atmungsstörungsereignisse die Anweisungen bei Ausführung durch den Prozessor das System dazu veranlassen, einen CPC-CVHR-Ereignistyp für jedes der Atmungsstörungsereignisse aus der Gruppe auszuwählen: hochfrequente Kopplung ohne CVHR (HFC), HFC mit CVHR (HFC_{CVHR}), niederfrequente Kopplung ohne CVHR (LFC), LFC mit CVHR (LFC_{CVHR}), REM ohne CVHR (REM), REM mit CVHR (REM_{CVHR}), Aufwachen ohne CVHR (WAKE), Aufwachen mit CVHR (WAKE_{CVHR}), erhöhtes breites Frequenzband mit niederfrequenter Kopplung ohne CVHR (eLFCBB), eLFCBB mit CVHR (eLFCBB_{CVHR}), erhöhtes schmales Frequenzband mit niederfrequenter Kopplung ohne CVHR (eLFCNB) und eLFCNB mit CVHR (eLFCNB_{CVHR}).

15. System nach Anspruch 14, wobei die Anweisungen bei Ausführung durch den Prozessor das System ferner dazu veranlassen, eine Gesamtanzahl von Ereignissen für jeden der Ereignistypen zu bestimmen, einschließend: HFC, HFC mit CVHR, LFC, LFC mit CVHR, REM, REM mit CVHR, Aufwachen, Aufwachen mit CVHR, eLFCBB, eLFCBB mit CVHR, eLFCNB und eLFCNB mit CVHR.

16. System nach Anspruch 15, wobei die Anweisungen bei Ausführung durch den Prozessor das System ferner veranlassen zum:
Bestimmen eines Schlafstörungsprävalenzmaßes auf der Basis der Gesamtanzahl der Atmungsstörungsereignisse;
Bestimmen eines Schlafstörungsschweregradmaßes auf der Basis einer Verteilung der Atmungsstörungsereignisse über die Ereignistypen, wobei die Verteilung auf der Gesamtanzahl von Ereignissen für jeden der Ereignistypen basiert ist; und
Bestimmen eines Maßes von Schlafapnoe auf der Basis des Schlafstörungsprävalenzmaßes und des Schlafstörungsschweregradmaßes.

17. System nach Anspruch 15, wobei die Anweisungen bei Ausführung durch den Prozessor das System ferner veranlassen zum:
Bestimmen einer durchschnittlichen Entsättigungsgrößenordnung für jeden der Ereignistypen für Ereignisse in dem Ereignistyp und einer durchschnittlichen Entsättigungsrate für Ereignisse in dem Ereignistyp;
Zugreifen auf einen Größenordnungsschwellenwert und einen Ratenschwellenwert; und
Bestimmen, ob die Person eine Hypopnoe oder eine Apnoe erfahren hat, auf der Basis eines Vergleichens der durchschnittlichen Entsättigungsgrößenordnung für jeden Ereignistyp mit dem Größenordnungsschwellenwert, und
Vergleichen der durchschnittlichen Entsättigungsrate für jeden Ereignistyp mit dem Ratenschwellenwert.

18. System nach Anspruch 17, wobei das Bestimmen, ob die Person eine Hypopnoe oder eine Apnoe erfahren hat, ferner auf mindestens einer von einer Gesamtdauer der Atmungsstörungsereignisse, einer durchschnittlichen Sauerstoffsättigung für die Atmungsstörungsereignisse und einer Verteilung der Atmungsstörungsereignisse über die Ereignistypen basiert.

## Revendications

1. Procédé d'évaluation du sommeil, comportant les étapes consistant à :
accéder à des données de saturation en oxygène enregistrées (203) comprenant des mesures de saturation en oxygène pour une personne au cours d'une période de temps ;
déterminer des événements de trouble respiratoire (206) pour la personne au cours de la période de temps en se basant sur les données de saturation en oxygène ;
accéder à des données de couplage cardio-pulmonaire enregistrées (209) obtenues en provenance de la personne pendant le sommeil, dans lequel les données de couplage cardio-pulmonaire sont corrélées dans le temps avec les données de saturation en oxygène au cours de la période de temps ; et
déterminer un type d'événement de couplage cardio-pulmonaire (212) pour chacun des événements de trouble respiratoire qui ont été déterminés en se basant sur les données de saturation en oxygène, en se basant sur les données de couplage cardio-pulmonaire correspondant dans le temps aux événements de trouble respiratoire qui ont été déterminés en se basant sur les données de saturation en oxygène.

2. Procédé selon la revendication 1, dans lequel l'étape consistant à déterminer les événements de trouble respiratoire (206) comprend les étapes consistant à :
identifier le début d'un événement de trouble respiratoire potentiel (301) par une diminution entre une première mesure de saturation en oxygène et une deuxième mesure de saturation en oxygène dans les données de saturation en oxygène, dans lequel la deuxième mesure de saturation en oxygène est temporellement postérieure à la première mesure de saturation en oxygène ; et
valider l'événement de trouble respiratoire potentiel (321) par au moins l'une des conditions comprenant :
des mesures successives de saturation en oxygène qui restent inchangées pendant une durée de plateau prédéterminée (323),
des mesures successives de saturation en oxygène qui vont en augmentant pendant une durée d'augmentation prédéterminée (326),
le fait de parvenir à une limite de durée prédéterminée depuis le début d'un événement de trouble respiratoire potentiel (329),
un taux de désaturation en oxygène de mesures successives de saturation en oxygène qui dépasse une limite de désaturation prédéterminée (331), ou
l'apparition d'une mesure de saturation en oxygène non valide (335),
dans lequel les mesures successives de saturation en oxygène sont temporellement postérieures à la deuxième mesure de saturation en oxygène.

3. Procédé selon la revendication 1, dans lequel l'étape consistant à déterminer les événements de trouble respiratoire comprend les étapes consistant à :
identifier un événement de trouble respiratoire potentiel par une durée au cours de laquelle les mesures de saturation en oxygène du SO₂ dans les données de saturation en oxygène sont inférieures à un seuil d'événement de SO₂ prédéterminé (506) ; et
déterminer comme quoi l'événement de trouble respiratoire potentiel n'est pas un événement de trouble respiratoire par au moins l'une des conditions comprenant :
la durée qui est inférieure à une durée minimale prédéterminée (515),
une amplitude de désaturation de SO₂ sur la durée qui est inférieure à un seuil minimum prédéterminé de désaturation de SO₂ (518), ou
l'événement de trouble respiratoire potentiel qui comprend un taux de désaturation initial de SO₂ qui est égal ou supérieur à un taux de désaturation maximum prédéterminé de SO₂ (521).

4. Procédé selon la revendication 1, comportant par ailleurs les étapes consistant à :
accéder à la variation cyclique enregistrée de données de fréquence cardiaque (612) obtenues en provenance de la personne ;
combiner les données de couplage cardio-pulmonaire et la variation cyclique de données de fréquence cardiaque (615) pour obtenir des données CPC-CVHR, dans lequel les données CPC-CVHR sont corrélées dans le temps avec les données de saturation en oxygène au cours de la période de temps ; et
déterminer un type d'événement CPC-CVHR (618) pour chacun des événements de trouble respiratoire en se basant sur les données CPC-CVHR correspondant dans le temps aux événements de trouble respiratoire.

5. Procédé selon la revendication 4, dans lequel l'étape consistant à déterminer le type d'événement CPC-CVHR (618) pour chacun des événements de trouble respiratoire comprend l'étape consistant à sélectionner, pour chacun des événements de trouble respiratoire, un type d'événement CPC-CVHR dans le groupe constitué par : un couplage haute fréquence sans CVHR (HFC), HFC avec CVHR (HFC_{CVHR}), un couplage basse fréquence sans CVHR (LFC), LFC avec CVHR (LFC_{CVHR}), REM sans CVHR (REM), REM avec CVHR (REM_{CVHR}), un éveil sans CVHR (WAKE), un éveil avec CVHR (WAKE_{CVHR}), un couplage basse fréquence élevé à large bande sans CVHR (eLFCBB), eLFCBB avec CVHR (eLFCBB_{CVHR}), un couplage basse fréquence élevé à bande étroite sans CVHR (eLFCNB), et eLFCNB avec CVHR (eLFCNB_{CVHR}).

6. Procédé selon la revendication 5, comportant par ailleurs l'étape consistant à déterminer un nombre total d'événements (621) pour chacun des types d'événements comprenant : HFC, HFC avec CVHR, LFC, LFC avec CVHR, REM, REM avec CVHR, éveil, éveil avec CVHR, eLFCBB, eLFCBB avec CVHR, eLFCNB et eLFCNB avec CVHR.

7. Procédé selon la revendication 6, comportant par ailleurs les étapes consistant à :
déterminer une mesure de prévalence de trouble du sommeil en se basant sur le nombre total d'événements de trouble respiratoire ;
déterminer une mesure de sévérité de trouble du sommeil (627) en se basant sur une distribution des événements de trouble respiratoire sur l'ensemble des types d'événements, la distribution étant basée sur le nombre total d'événements pour chacun des types d'événements ; et
déterminer une mesure de l'apnée du sommeil (630) en se basant sur la mesure de prévalence de trouble du sommeil et sur la mesure de sévérité de trouble du sommeil.

8. Procédé selon la revendication 6, comportant par ailleurs les étapes consistant à :
déterminer, pour chacun des types d'événements, une amplitude moyenne de désaturation (633) pour des événements du type d'événement et un taux moyen de désaturation pour des événements du type d'événement ;
accéder à un seuil d'amplitude et à un seuil de taux (636) ; et
déterminer si la personne a souffert d'hypopnée ou d'apnée (639) en se basant sur une comparaison de l'amplitude moyenne de désaturation pour chaque type d'événement par rapport au seuil d'amplitude, et
comparer le taux moyen de désaturation pour chaque type d'événement par rapport au seuil de taux.

9. Procédé selon la revendication 8, dans lequel l'étape consistant à déterminer si la personne a souffert d'hypopnée ou d'apnée est basée par ailleurs sur au moins une donnée parmi une durée totale des événements de trouble respiratoire, une saturation en oxygène moyenne pour les événements de trouble respiratoire, et une distribution des événements de trouble respiratoire sur l'ensemble des types d'événements.

10. Système (900) d'évaluation du sommeil, comportant :
un ou plusieurs processeurs (904) ; et
au moins une mémoire (908, 910) stockant des instructions qui, quand elles sont exécutées par lesdits un ou plusieurs processeurs, amènent le système à :
accéder à des données de saturation en oxygène comprenant des mesures de saturation en oxygène pour une personne au cours d'une période de temps ;
déterminer des événements de trouble respiratoire pour la personne au cours de la période de temps en se basant sur les données de saturation en oxygène ;
accéder à des données de couplage cardio-pulmonaire pour la personne pendant le sommeil, dans lequel les données de couplage cardio-pulmonaire sont corrélées dans le temps avec les données de saturation en oxygène au cours de la période de temps ; et
déterminer un type d'événement de couplage cardio-pulmonaire pour chacun des événements de trouble respiratoire qui ont été déterminés en se basant sur les données de saturation en oxygène, en se basant sur les données de couplage cardio-pulmonaire correspondant dans le temps aux événements de trouble respiratoire qui ont été déterminés en se basant sur les données de saturation en oxygène.

11. Système selon la revendication 10, dans lequel, lors de la détermination des événements de trouble respiratoire, les instructions, quand elles sont exécutées par le processeur, amènent le système à :
identifier le début d'un événement de trouble respiratoire potentiel par une diminution entre une première mesure de saturation en oxygène et une deuxième mesure de saturation en oxygène dans les données de saturation en oxygène, dans lequel la deuxième mesure de saturation en oxygène est temporellement postérieure à la première mesure de saturation en oxygène ; et
valider l'événement de trouble respiratoire potentiel par au moins l'une des conditions comprenant :
des mesures successives de saturation en oxygène qui restent inchangées pendant une durée de plateau prédéterminée,
des mesures successives de saturation en oxygène qui vont en augmentant pendant une durée d'augmentation prédéterminée,
le fait de parvenir à une limite de durée prédéterminée depuis le début d'un événement de trouble respiratoire potentiel,
un taux de désaturation en oxygène de mesures successives de saturation en oxygène qui dépasse une limite de désaturation prédéterminée, ou
l'apparition d'une mesure de saturation en oxygène non valide,
dans lequel les mesures successives de saturation en oxygène sont temporellement postérieures à la deuxième mesure de saturation en oxygène.

12. Système selon la revendication 10, dans lequel, lors de la détermination des événements de trouble respiratoire, les instructions, quand elles sont exécutées par le processeur, amènent le système à :
identifier un événement de trouble respiratoire potentiel par une durée au cours de laquelle les mesures de saturation en oxygène du SO₂ dans les données de saturation en oxygène sont inférieures à un seuil prédéterminé d'événement de SO₂ ; et
déterminer comme quoi l'événement de trouble respiratoire potentiel n'est pas un événement de trouble respiratoire par au moins l'une des conditions comprenant :
la durée qui est inférieure à une durée minimale prédéterminée,
une amplitude de désaturation de SO₂ sur la durée qui est inférieure à un seuil minimum prédéterminé de désaturation de SO₂, ou
l'événement de trouble respiratoire potentiel qui comprend un taux de désaturation initial de SO₂ qui est égal ou supérieur à un taux de désaturation maximum prédéterminé de SO₂.

13. Système selon la revendication 10, dans lequel les instructions, quand elles sont exécutées par le processeur, amènent par ailleurs le système à :
accéder à la variation cyclique de données de fréquence cardiaque pour la personne ;
combiner les données de couplage cardio-pulmonaire et la variation cyclique de données de fréquence cardiaque pour obtenir des données CPC-CVHR, dans lequel les données CPC-CVHR sont corrélées dans le temps avec les données de saturation en oxygène au cours de la période de temps ; et
déterminer un type d'événement CPC-CVHR pour chacun des événements de trouble respiratoire en se basant sur les données CPC-CVHR correspondant dans le temps aux événements de trouble respiratoire.

14. Système selon la revendication 13, dans lequel, lors de la détermination du type d'événement CPC-CVHR pour chacun des événements de trouble respiratoire, les instructions, quand elles sont exécutées par le processeur, amènent le système à sélectionner, pour chacun des événements de trouble respiratoire, un type d'événement CPC-CVHR dans le groupe constitué par : un couplage haute fréquence sans CVHR (HFC), HFC avec CVHR (HFC_{CVHR}), un couplage basse fréquence sans CVHR (LFC), LFC avec CVHR (LFC_{CVHR}), REM sans CVHR (REM), REM avec CVHR (REM_{CVHR}), un éveil sans CVHR (WAKE), un éveil avec CVHR (WAKE_{CVHR}), un couplage basse fréquence élevé à large bande sans CVHR (eLFCBB), eLFCBB avec CVHR (eLFCBB_{CVHR}), un couplage basse fréquence élevé à bande étroite sans CVHR (eLFCNB), et eLFCNB avec CVHR (eLFCNB_{CVHR}).

15. Système selon la revendication 14, dans lequel les instructions, quand elles sont exécutées par le processeur, amènent par ailleurs le système à déterminer un nombre total d'événements pour chacun des types d'événements comprenant : HFC, HFC avec CVHR, LFC, LFC avec CVHR, REM, REM avec CVHR, éveil, éveil avec CVHR, eLFCBB, eLFCBB avec CVHR, eLFCNB et eLFCNB avec CVHR.

16. Système selon la revendication 15, dans lequel les instructions, quand elles sont exécutées par le processeur, amènent par ailleurs le système à :
déterminer une mesure de prévalence de trouble du sommeil en se basant sur le nombre total d'événements de trouble respiratoire ;
déterminer une mesure de sévérité de trouble du sommeil en se basant sur une distribution des événements de trouble respiratoire sur l'ensemble des types d'événements, la distribution étant basée sur le nombre total d'événements pour chacun des types d'événements ; et
déterminer une mesure de l'apnée du sommeil en se basant sur la mesure de prévalence de trouble du sommeil et sur la mesure de sévérité de trouble du sommeil.

17. Système selon la revendication 15, dans lequel les instructions, quand elles sont exécutées par le processeur, amènent par ailleurs le système à :
déterminer, pour chacun des types d'événements, une amplitude moyenne de désaturation pour des événements du type d'événement et un taux moyen de désaturation pour des événements du type d'événement ;
accéder à un seuil d'amplitude et à un seuil de taux ; et
déterminer si la personne a souffert d'hypopnée ou d'apnée en se basant sur une comparaison de l'amplitude moyenne de la désaturation pour chaque type d'événement par rapport au seuil d'amplitude, et
comparer le taux moyen de désaturation pour chaque type d'événement par rapport au seuil de taux.

18. Système selon la revendication 17, dans lequel l'étape consistant à déterminer si la personne a souffert d'hypopnée ou d'apnée est basée par ailleurs sur au moins une donnée parmi une durée totale des événements de trouble respiratoire, une saturation en oxygène moyenne pour les événements de trouble respiratoire, et une distribution des événements de trouble respiratoire sur l'ensemble des types d'événements.
